# EUROPÄISCHE PATENTSCHRIFT

(11) **EP 1 769 079 B1**
(45) Veröffentlichungstag und Bekanntmachung des Hinweises auf die Patenterteilung: **28.06.2017**
(21) Anmeldenummer: 05772114.4
(22) Anmeldetag: 06.07.2005
(51) Int. Cl.: C08G 18/67, C12P 7/62, C07C 69/54, C09D 175/16, C09D 4/00, C08F 220/28

(54) **ENZYMATISCHE HERSTELLUNG VON (METH)ACRYLSÄUREESTERN**
ENZYMATIC PRODUCTION OF (METH)ACRYLIC ACID ESTERS
PRODUCTION ENZYMATIQUE D'ESTERS D'ACIDE METHACRYLIQUE

(30) Priorität: 09.07.2004 DE 102004033555; 11.05.2005 US 679662 P
(43) Veröffentlichungstag der Anmeldung: 04.04.2007
(73) Patentinhaber: BASF SE, 67056 Ludwigshafen am Rhein (DE)
(72) Erfinder: HÄRING, Dietmar, 69198 Schriesheim (DE); MEISENBURG, Uwe, 68161 Mannheim (DE); HAUER, Bernhard, 67136 Fussgönheim (DE)
(86) Internationale Anmeldenummer: PCT/EP2005/007276
(87) Internationale Veröffentlichungsnummer: WO 2006/005491

(56) Entgegenhaltungen:
- EP-A- 0 675 141
- WO-A-2004/048585
- HAJJAR A B ET AL: "PREPARATION OF MONOMERIC ACRYLIC ESTER INTERMEDIATES USING LIPASE CATALYSED TRANSESTERIFICATIONS IN ORGANIC SOLVENTS" BIOTECHNOLOGY LETTERS, KEW, SURREY, GB, Bd. 12, Nr. 11, 1990, Seiten 825-830, XP008031024 ISSN: 0141-5492 in der Anmeldung erwähnt
- GOEDE ET AL.: "Selective lipase-catalyzed esterification of alkyl glycosides" BIOCATALYSIS, Bd. 9, 1994, Seiten 145-155, XP008055677 in der Anmeldung erwähnt

## Beschreibung

Die vorliegende Erfindung betrifft ein Verfahren zur Herstellung von teilveresterten (Meth)acrylsäureestern von Polyalkoholen mit unterschiedlichen Hydroxygruppen.

Die Herstellung von (Meth)acrylsäureestern erfolgt zumeist durch säure- oder basenkatalysierte Veresterung von (Meth)acrylsäure oder Umesterung von anderen (Meth)acrylsäureestern mit Alkoholen.

Teilveresterte (Meth)acrylsäureester von Polyalkoholen mit unterschiedlichen Hydroxygruppen lassen sich durch eine Ver- oder Umesterung in der Regel nicht gezielt herstellen, da statistische Gemische erhalten werden.

Bei basenkatalysierter Umesterung oder anderen Synthesen entstehen oft komplexe und bisweilen gefärbte Produktgemische. Zur Entfernung von Färbungen und unumgesetzten Reaktanden müssen die Produktgemische durch aufwendige alkalische Wäschen aufgearbeitet werden.

Die unveröffentlichte deutsche Patentanmeldung mit dem Aktenzeichen 10308504.1 offenbart ein Verfahren zur Herstellung teilacrylierter Polyole durch Schützen von OH-Gruppen mit Hilfe von Acetal- oder Ketalgruppen, enzymatischer (Meth)Acrylierung der freien OH-Gruppen und gegebenenfalls anschließender Entschützung.

Nachteilig an diesem Prozeß ist, daß zusätzliche Schritte erforderlich sind zum Einbringen und Entfernen der Schutzgruppen.

Die Herstellung von (Meth)acrylsäureestern durch eine enzymatische Ver- oder Umesterung ist bekannt.

Kumar und Gross beschreiben in J. Am. Chem. Soc. 2002, 124, 1850-1851 die Lipasekatalysierte Umsetzung von Isopropyliden-geschützten Zuckern durch Umsetzung mit Vinylmethacrylat. Eine vollständige Umsetzung wird erreicht durch das spezielle Edukt Vinylmethacrylat, da freigesetzter Vinylalkohol dem Reaktionsgleichgewicht als Acetaldehyd entzogen wird. Nachteilig an diesem Verfahren ist, daß Vinylmethacrylat als Spezialmonomer teuer und kommerziell nur in kleinen Mengen verfügbar ist.

A. T. J. W. de Goede et al. beschreiben in Biocatalysis, 1994, 9, 145 - 155 die Umesterung von α-O-Octyl-Glucosid mit Ethylacrylat zum 6-O-Acrylester in Gegenwart von Lipasen. Nachteilig an diesem Verfahren ist, daß es auf Glucoside und glykosidische Bindungen beschränkt ist und empfindlich auf sterische Einflüsse im Glucosid reagiert. Zudem wurden höher acrylierte Produkte infolge von unselektiven Nebenreaktionen erhalten.

EP-A1 999 229 beschreibt die enzymatische Ver- und Umesterung von Polyoxyalkylenen mit (Meth)Acrylsäure und (Meth)Acrylsäureestern.

Aus WO 03/042227 ist die Lipase-katalysierte Umesterung von Alkylacrylaten mit Zuckern bekannt.

Hajjar et al. beschreiben in Biotechnol. Lett. 1990, 12, 825-830 die enzymatische Umesterung von cyclischen und offenkettigen Alkandiolen mit Ethylacrylat mit einer Lipase aus *Chromobacterium viscosum.* Die Reaktionen laufen bei einem 18-fachen molaren Überschuß des Alkylacrylats gegenüber dem Diol in einem lösungsmittelfreien System ab. Es entstehen Mischungen aus Mono- und Diacrylaten.

Dabei wurden jeweils 5 mmol Diol in 10 ml Ethylacrylat (92 mmol) mit 100 mg Lipase aus Chromobacterium viscosum bei 30 °C umgesetzt.

Bei der Acrylierung von Diolen mit zwei primären OH-Gruppen (zB 1,6-Hexandiol) war das Diol nach 7 d zu >95 % umgesetzt. Es entstand eine Mischung aus Mono- und Diacrylaten, die sich je nach Reaktionszeit >80 % Diacrylat enthielt.

Bei der Acrylierung von Diolen mit zwei sekundären OH-Gruppen (zB 2,5-Hexandiol) war die Reaktionszeit deutlich höher: Selbst nach 14 Tagen war das Diol nur zu etwa 50 % umgesetzt. Es entstand eine Acrylatmischung aus Mono- und Diacrylaten, die 70 % Mono- und 30 % Diacrylat enthielt.

Bei der Acrylierung von Diolen mit einer primären und einer sekundären OH-Gruppe (zB 1,2-Hexandiol) war das Diol nach 7 d erst zu etwa 85 % umgesetzt. Es entstand eine Mischung aus Mono- und Diacrylaten, die nach 14 Tagen Reaktionszeit 5 % Diacrylat enthielt. Der Gehalt an Diacrylat stieg im Laufe der Reaktionszeit kontinuierlich an.

Somit kann unter diesen Reaktionsbedingungen keine ausreichende Selektivität von Mono- zu Diacrylierungsprodukt erhalten werden, man findet lediglich die übliche, mäßige Selektivität zwischen primären und sekundären Alkohogruppen.

US 5240835 beschreibt die Umesterung von Alkylacrylaten mit Alkoholen unter Katalyse eines Biokatalysators aus *Corynebacterium oxydans.* Beispielhaft wird dort die Reaktion von einem 96-fachen molaren Überschuß Ethylacrylat mit 2,2-Dimethyl-1,3-propandiol aufgeführt. Lediglich 21 % Ausbeute wurden nach 3 Tagen bei 30 °C erhalten.

Athawale und Manjrekar (Tetrahedron Lett. 2001, 42 4541-4543) beschreiben die Lipase-katalysierte Acrylierung von Alkaloiden mittels 2,3-Butandion-monooximacrylat. Das Monomer wurde polymerisiert und zur Induktion von enantioselektiven Michael-Addition eingesetzt.

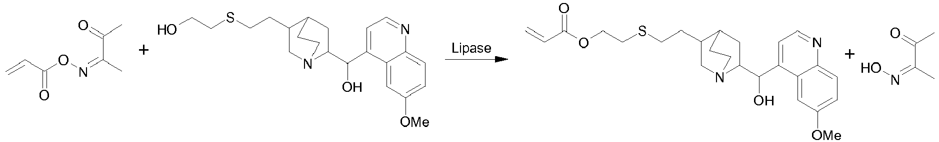

Athawale und Gaonkar (Macromolecules 1999,32, 6065-6068) beschreiben die Lipasekatalysierte Acrylierung von 2-Phenylethanolen mittels 2,3-Butandion-monooximacrylat. Das Monomer wurde anschließend polymerisiert.

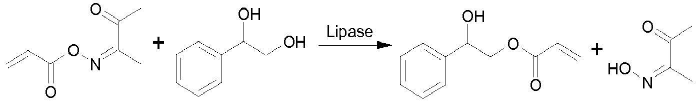

Ghogare und Kumar (J. Chem. Soc. 1989, 1533-1535) beschreiben die Lipasekatalysierte Acrylierung von verschiedenen Alkoholen, u.a. 2-Ethylhexan-1,3-diol, mit einem aktivierten 2,3-Butandion-monooximacrylat.

Nachteilig an diesen Reaktionen ist die Notwendigkeit zur Verwendung von aktivierten Acrylaten, die teuer und industriell schlecht verfügbar sind.

WO 2004/048585 beschreibt die enzymatische Synthese Polyolacrylaten mit wenigstens 3, bevorzugt 3 bis 10, Hydroxylgruppen, die gleichartig sein können.

Aufgabe der vorliegenden Erfindung war es, ein Verfahren zur Verfügung zu stellen, mit dem teilveresterte (Meth)acrylsäureester von Polyalkoholen mit unterschiedlichen Hydroxygruppen in hohen Umsätzen und hohen Selektivitäten bzgl. der Reaktion an den unterschiedlichen Hydroxygruppen und hohen Reinheiten aus einfachen Edukten herstellbar sind. Die Synthese sollte unter milden Bedingungen ablaufen, so daß Produkte mit einer niedrigen Farbzahl und hoher Reinheit resultieren. Zudem soll auf Schutzgruppen verzichtet werden, ebenso auf den Einsatz aktivierter (Meth)Acrylsäurederivate, wie beispielsweise Monooxime oder Vinyl(meth)acrylat.

Die Aufgabe wurde gelöst durch ein Verfahren zur Herstellung von teilveresterten (Meth)acrylsäureestern (F) von zweiwertigen Polyalkoholen (C), die unterschiedliche Hydroxygruppen enthalten, dadurch gekennzeichnet, dass man entweder
(1) mindestens einen Polyalkohol (C¹) mit einer primären Hydroxygruppe und einer sekundären Hydroxygruppe, oder
(2) mindestens einen Polyalkohol (C²) mit einer primären Hydroxyguppe und einer tertiären Hydroxygruppe,
in Gegenwart mindestens eines Enzyms (E) mit (Meth)acrylsäure verestert oder mindestens einem (Meth)acrylsäureester (D) umestert, wobei der Anteil an mehrfach (meth)acryliertem Produkt nicht mehr als 5 mol%, bezogen auf den Polyalkohol (C), beträgt,
wobei der Wasseranteil im Reaktionsansatz bei 0-10 Vol% liegt, dadurch gekennzeichnet, dass es sich bei dem Polyalkohol (C) um einen der Formel II 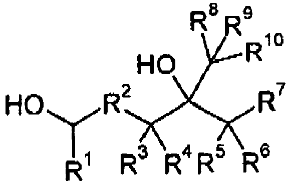 oder Formel III handelt 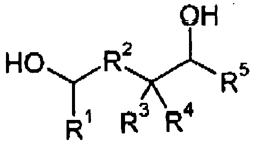 worin,
R¹ und R³ bis R¹⁰ unabhängig voneinander ausgewählt sind aus der Gruppe umfassend Wasserstoff, Methyl, Ethyl, n-Propyl, n-Butyl, n-Hexyl, n-Heptyl, n-Octyl, n-Decyl, n-Dodecyl, n-Tetradecyl, n-Hexadecyl, n-Octadecyl, n-Eicosyl, 2-Ethylhexyl, Cyclopentyl, Cyclohexyl, Cyclooctyl, Cyclododecyl, Phenyl, Naphthyl oder Benzyl, und
R² eine Einfachbindung, C₁-C₂₀-Alkylen, C₅-C₁₂-Cycloalkylen, C₆-C₁₂-Arylen oder durch ein- oder mehrere Sauerstoff- und/oder Schwefelatome und/oder ein oder mehrere substituierte oder unsubstituierte Iminogruppen und/oder durch eine o-der mehrere Cycloalkyl-, -(CO)-, -O(CO)O-, -(NH)(CO)O-, -O(CO)(NH)-, -O(CO)-oder -(CO)O-Gruppen unterbrochenes C2-C20-Alkylen, wobei die genannten Reste jeweils durch Aryl, Alkyl, Aryloxy, Alkyloxy, Heteroatome und/oder Heterocyclen substituiert sein können,
bedeuten,
wobei
für den Fall (2) in Anspruch 1 in Formel II R1 Wasserstoff ist und mindestens einer der Reste R3 bis R10 ungleich Wasserstoff ist.

Offenbart ist auch ein Verfahren zur Herstellung von teilveresterten (Meth)acrylsäureestern (F) von mindestens zweiwertigen Polyalkoholen (C), die unterschiedliche Hydroxygruppen enthalten, dadurch gekennzeichnet, daß man entweder
(1) mindestens einen Polyalkohol (C₁) mit mindestens einer primären Hydroxyguppe und mindestens einer sekundären Hydroxyguppe,
   oder
(2) mindestens einen Polyalkohol (C₂) mit mindestens einer primären Hydroxyguppe und mindestens einer tertiären Hydroxyguppe,
   oder
(3) mindestens einen Polyalkohol (C₃) mit mindestens einer sekundären Hydroxyguppe und mindestens einer tertiären Hydroxyguppe,
   wobei die höhersubstituierte Hydroxygruppe in Position β mindestens einen Alkyl-, Cycloalkyl-, Aryl- oder Aralkylrest trägt,
   oder
(4) mindestens einen Polyalkohohol (C₄) mit mindestens zwei primären Hydroxygruppen, von denen mindestens eine in Position β mindestens einen und mindestens eine in Position β keinen Alkyl-, Cycloalkyl-, Aryl- oder Aralkylrest trägt,
   oder
(5) mindestens einen Polyalkohohol (C₅) mit mindestens zwei sekundären Hydroxygruppen, von denen mindestens eine in Position β mindestens einen und mindestens eine in Position β keinen Alkyl-, Cycloalkyl-, Aryl- oder Aralkylrest trägt, in Gegenwart mindestens eines Enzyms (E) mit (Meth)acrylsäure verestert oder mindestens einem (Meth)acrylsäureester (D) umestert.

Mit Hilfe des erfindungsgemäßen Verfahrens ist die Herstellung teilveresterten (Meth)acrylsäureester (F) in hoher chemischer und Raum-Zeit-Ausbeute und unter milden Bedingungen mit guten Farbzahlen unter Verzicht auf Schutzgruppenoperationen und unter Einsatz einfacher Ausgangsstoffe möglich.

(Meth)acrylsäure steht in dieser Schrift für Methacrylsäure und Acrylsäure, bevorzugt für Acrylsäure. Geeignete Polyalkohole (C) sind mindestens zweiwertige, bevorzugt zwei- bis zehnwertige, besonders bevorzugt zwei- bis sechswertige, ganz besonders bevorzugt zwei- bis vierwertige, insbesondere zwei- bis dreiwertige und speziell zweiwertige Alkohole.

Diese erfindungsgemäß geeigneten Polyalkohole (C) tragen unterscheidbare Hydroxygruppen, d.h. sie weisen erfindungsgemäß mindestens eine niedrigersubstituierte und mindestens eine höhersubstituierte Hydroxygruppe im selben Molekül auf. Durch die unterschiedliche Umgebung der Hydroxygruppen werden diese in einer enzymatischen (Meth)acrylierung unterscheidbar, so daß man gemäß dem erfindungsgemäßen Verfahren eine höhere Selektivität erreicht als mit den aus dem Stand der Technik bekannten Verfahren.

Mit niedriger- und höhersubstituiert ist damit die Anzahl der Substituenten an dem Kohlenstoffatom gemeint, mit dem die jeweilige Hydroxygruppe verbunden ist, also am α-Kohlenstoffatom (siehe unten).

Niedrigersubstituierte Hydroxygruppen sind beispielsweise primäre oder sekundäre Hydroxygruppen, höhersubstituierte Hydroxygruppen sind beispielsweise sekundäre oder tertiäre Hydroxygruppen. Dabei bezieht sich der Substitutionsgrad auf die Anzahl an Nicht-Wasserstoffatomen, die mit dem Kohlenstoffatom verbunden sind, das mit der betrachteten Hydroxygruppe verbunden ist.

Dabei weisen die Polyalkohole (C) mindestens eine niedrigersubstituierte Hydroxygruppe auf, bevorzugt 1 bis 6, besonders bevorzugt 1 bis 4, ganz besonders bevorzugt 1 bis 3 insbesondere 1 bis 2 und speziell eine sowie mindestens eine höhersubstituierte Hydroxygruppe, bevorzugt 1 bis 6, besonders bevorzugt 1 bis 4, ganz besonders bevorzugt 1 bis 3 insbesondere 1 bis 2 und speziell eine.

Zusätzliches erfindungsgemäßes Merkmal ist das Vorhandensein mindestens eines Alkyl-, Cycloalkyl-, Aryl- oder Aralkylrestes in Position β zur höher substituierten Hydroxygruppe, bevorzugt ist mindestens ein Alkyl-, Cycloalkyl-, Aryl- oder Aralkylrest in Position β.

Dabei ist die allgemeine Konvention die, daß das Kohlenstoffatom, mit dem die betrachtete Hydroxygruppe verbunden ist, das α-Kohlenstoffatom ist, das übernächste das β-Kohlenstoffatom und danach das γ-Kohlenstoffatom, wie beispielsweise im Folgenden schematisch dargestellt (eventuelle Substituenten sind nicht gezeichnet):

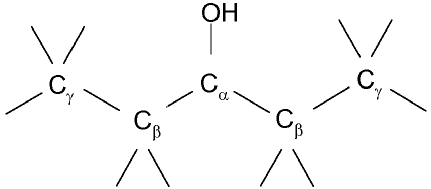

Offenbarte Polyalkohole sind
entweder
(1) Polyalkohole (C₁) mit mindestens einer primären Hydroxyguppe und mindestens einer sekundären Hydroxyguppe,
   oder
(2) Polyalkohole (C₂) mit mindestens einer primären Hydroxyguppe und mindestens einer tertiären Hydroxyguppe,
   oder
(3) Polyalkohole (C₃) mit mindestens einer sekundären Hydroxyguppe und mindestens einer tertiären Hydroxyguppe,
   oder
(4) Polyalkohohole (C₄) mit mindestens zwei primären Hydroxygruppen, von denen mindestens eine in Position β mindestens einen und mindestens eine in Position β keinen Alkyl-, Cycloalkyl-, Aryl- oder Aralkylrest trägt,
   oder
(5) Polyalkohohole (C₅) mit mindestens zwei sekundären Hydroxygruppen, von denen mindestens eine in Position β mindestens einen und mindestens eine in Position β keinen Alkyl-, Cycloalkyl-, Aryl- oder Aralkylrest trägt.

Offenbart sind Polyalkohole (C) der Formeln I 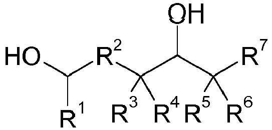 und Formel II, 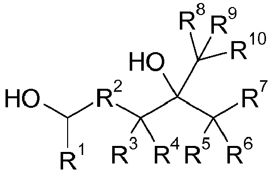 und Formel III, 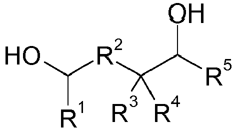 worin
R¹ und R³ bis R¹⁰ jeweils unabhängig voneinander Wasserstoff, C₁- C₁₈-Alkyl, gegebenenfalls durch ein oder mehrere Sauerstoff- und/oder Schwefelatome und/oder ein oder mehrere substituierte oder unsubstituierte Iminogruppen unterbrochenes C₂ - C₁₈-Alkyl, C₆ - C₁₂-Aryl, C₅ - C₁₂-Cycloalkyl oder einen fünf- bis sechsgliedrigen, Sauerstoff-, Stickstoff- und/oder Schwefelatome aufweisenden Heterocyclus, wobei die genannten Reste jeweils durch Aryl, Alkyl, Aryloxy, Alkyloxy, Heteroatome und/oder Heterocyclen substituiert sein können, und
R² eine Einfachbindung, C₁-C₂₀-Alkylen, C₅-C₁₂-Cycloakylen, C₆-C₁₂-Arylen oder durch ein- oder mehrere Sauerstoff- und/oder Schwefelatome und/oder ein oder mehrere substituierte oder unsubstituierte Iminogruppen und/oder durch eine oder mehrere Cycloalkyl-, -(CO)-, -O(CO)O-, -(NH)(CO)O-, -O(CO)(NH)-, -O(CO)- oder -(CO)O-Gruppen unterbrochenes C₂-C₂₀-Alkylen, wobei die genannten Reste jeweils durch Aryl, Alkyl, Aryloxy, Alkyloxy, Heteroatome und/oder Heterocyclen substituiert sein können, bedeuten,
wobei
für den Fall (1) in Formel I R¹ Wasserstoff ist und mindestens einer der Reste R³ bis R⁷ ungleich Wasserstoff ist,
für den Fall (2) in Formel II R¹ Wasserstoff ist und mindestens einer der Reste R³ bis R¹⁰ ungleich Wasserstoff ist und
für den Fall (3) in Formel II R¹ ungleich Wasserstoff und mindestens einer der Reste R³ bis R¹⁰ ungleich Wasserstoff ist,
für den Fall (4) in Formel III R¹ und R⁵ gleich Wasserstoff sind und mindestens einer der Reste R³ und R⁴ ungleich Wasserstoff ist und
für den Fall (5) in Formel III R¹ und R⁵ ungleich Wasserstoff sind und mindestens einer der Reste R³ und R⁴ ungleich Wasserstoff ist.

Bevorzugt weisen die Reste R¹ bis R¹⁰ keine weiteren Hydroxy- und/oder Aminogruppen auf, besonders bevorzugt weisen die Reste R¹ bis R¹⁰ keine Hydroxy- und/oder Amino- und/oder Ester- und/oder Amidgruppen auf und ganz besonders bevorzugt weisen die Reste R¹ bis R¹⁰ keine weiteren Sauerstoff- und/oder Stickstoffatome auf. Bevorzugt handelt es sich bei den Resten R¹ und R³ bis R¹⁰ um Wasserstoff oder Kohlenwasserstoffe, d.h. sie enthalten ausschließlich Kohlenstoff und Wasserstoff.

Besonders bevorzugt handelt es sich bei dem Rest R² und R³ bis R¹⁰ um eine Einfachbindung oder einen Kohlenwasserstoff.

In den obigen Definitionen bedeuten
gegebenenfalls durch Aryl, Alkyl, Aryloxy, Alkyloxy, Heteroatome und/oder Heterocyclen substituiertes C₁-C₂₀-Alkylen beispielsweise Methylen, 1,1-Ethylen, 1,2-Ethylen, 1,1-Propylen, 1,2-Propylen, 1,3-Propylen, 1,1-Butylen, 1,2-Butylen, 1,3-Butylen, 1,4-Butylen, 1,6-Hexylen, 2-Methyl-1,3-Propylen, 2-Ethyl-1,3-Propylen, 2,2-Dimethyl-1,3-Propylen, 2,2-Dimethyl-1,4-butylen,
gegebenenfalls durch Aryl, Alkyl, Aryloxy, Alkyloxy, Heteroatome und/oder Heterocyclen substituiertes C₅-C₁₂-Cycloalkylen beispielsweise Cyclopropylen, Cyclopentylen, Cyclohexylen, Cyclooctylen, Cyclododecylen,
gegebenenfalls durch Aryl, Alkyl, Aryloxy, Alkyloxy, Heteroatome und/oder Heterocyclen substituiertes, durch ein- oder mehrere Sauerstoff- und/oder Schwefelatome und/oder ein oder mehrere substituierte oder unsubstituierte Iminogruppen und/oder durch eine oder mehrere Cycloalkyl-, -(CO)-, -O(CO)O-, -(NH)(CO)O-, -O(CO)(NH)-, -O(CO)- oder -(CO)O-Gruppen unterbrochenes C₂-C₂₀-Alkylen beispielsweise 1-Oxa-1,3-propylen, 1,4-Dioxa-1,6-hexylen, 1,4,7-Trioxa-1,9-nonylen, 1-Oxa-1,4-butylen, 1,5-Dioxa-1,8-octylen, 1-Oxa-1,5-pentylen, 1-Oxa-1,7-heptylen, 1,6-Dioxa-1,10-decylen, 1-Oxa-3-methyl-1,3-propylen, 1-Oxa-3-methyl-1,4-butylen, 1-Oxa-3,3-dimethyl-1,4-butylen, 1-Oxa-3,3-dimethyl-1,5-pentylen, 1,4-Dioxa-3,6-dimethyl-1,6-hexylen, 1-Oxa-2-methyl-1,3-propylen, 1,4-Dioxa-2,5-dimethyl-1,6-hexylen, 1-Oxa-1,5-pent-3-enylen, 1-Oxa-1,5-pent-3-inylen, 1,1-, 1,2-, 1,3- oder 1,4-Cyclohexylen, 1,2- oder 1,3-Cyclopentylen, 1,2-, 1,3- oder 1,4-Phenylen, 4,4'-Biphenylen, 1,4-Diaza-1,4-butylen, 1-Aza-1,3-propylen, 1,4,7-Triaza-1,7-heptylen, 1,4-Diaza-1,6-hexylen, 1,4-Diaza-7-oxa-1,7-heptylen, 4,7-Diaza-1-oxa-1,7-heptylen, 4-Aza-1-oxa-1,6-hexylen, 1-Aza-4-oxa-1,4-butylen, 1-Aza-1,3-propylen, 4-Aza-1-oxa-1,4-butylen, 4-Aza-1,7-dioxa-1,7-heptylen, 4-Aza-1-oxa-4-methyl-1,6-hexylen, 4-Aza-1,7-dioxa-4-methyl-1,7-heptylen, 4-Aza-1,7-dioxa-4-(2'-hydroxyethyl)-1,7-heptylen, 4-Aza-1-oxa-(2'-hydroxyethyl)-1,6-hexylen oder 1,4-Piperazinylen,
gegebenenfalls durch Aryl, Alkyl, Aryloxy, Alkyloxy, Heteroatome und/oder Heterocyclen substituiertes C₆-C₁₂-Arylen beispielsweise 1,2-, 1,3- oder 1,4-Phenylen, 4,4'-Biphenylen, Toluylen oder Xylylen,
C₁-C₁₈-Alkyl oder gegebenenfalls durch ein oder mehrere Sauerstoff- und/oder Schwefelatome und/oder ein oder mehrere substituierte oder unsubstituierte Iminogruppen unterbrochenes C₂ - C₁₈-Alkyl beispielsweise beispielsweise Methyl, Ethyl, Propyl, Isopropyl, n-Butyl, sec-Butyl, tert.-Butyl, Pentyl, Hexyl, Heptyl, Octyl, 2-Etylhexyl, 2,4,4-Trimethylpentyl, Decyl, Dodecyl, Tetradecyl, Hexadecyl, Octadecyl, 1,1-Dimethylpropyl, 1,1-Dimethylbutyl, 1,1,3,3-Tetramethylbutyl, Benzyl, 1-Phenylethyl, 2-Phenylethyl, α,α-Dimethylbenzyl, Benzhydryl, p-Tolylmethyl,1-(p-Butylphenyl)-ethyl, p-Chlorbenzyl, 2,4-Dichlorbenzyl, p-Methoxybenzyl, m-Ethoxybenzyl, 2-Cyanoethyl, 2-Cyanopropyl, 2-Methoxycarbonethyl, 2-Ethoxycarbonylethyl, 2-Butoxycarbonylpropyl, 1,2-Di-(methoxycarbonyl)-ethyl, 2-Methoxyethyl, 2-Ethoxyethyl, 2-Butoxyethyl, Diethoxymethyl, Diethoxyethyl, 1,3-Dioxolan-2-yl, 1,3-Dioxan-2-yl, 2-Methyl-1,3-dioxolan-2-yl, 4-Methyl-1,3-dioxolan-2-yl, 2-Isopropoxyethyl, 2-Butoxypropyl, 2-Octyloxyethyl, Chlormethyl, 2-Chlorethyl, Trichlormethyl, Trifluormethyl, 1,1-Dimethyl-2-chlorethyl, 2-Methoxyisopropyl, 2-Ethoxyethyl, Butylthiomethyl, 2-Dodecylthioethyl, 2-Phenylthioethyl, 2,2,2-Trifluorethyl, 2-Phenoxyethyl, 2-Phenoxypropyl, 3-Phenoxypropyl, 4-Phenoxy-butyl, 6-Phenoxyhexyl, 2-Methoxyethyl, 2-Methoxypropyl, 3-Methoxypropyl, 4-Methoxybutyl, 6-Methoxyhexyl, 2-Ethoxyethyl, 2-Ethoxypropyl, 3-Ethoxypropyl, 4-Ethoxybutyl oder 6-Ethoxyhexyl, und bevorzugt Methyl, Ethyl, Propyl, Isopropyl, n-Butyl, sec-Butyl, tert.-Butyl, Pentyl, Hexyl, Heptyl, Octyl, 2-Etylhexyl, 2,4,4-Trimethylpentyl, Decyl, Dodecyl, Tetradecyl, Hexadecyl, Octadecyl, 1,1-Dimethylpropyl, 1,1-Dimethylbutyl, 1,1,3,3-Tetramethylbutyl, Benzyl, 1-Phenylethyl, 2-Phenylethyl, α,α-Dimethylbenzyl, Benzhydryl, p-Tolylmethyl,1-(p-Butylphenyl)-ethyl, p-Chlorbenzyl, 2,4-Dichlorbenzyl, 2-Cyanoethyl, 2-Cyanopropyl, Chlormethyl, 2-Chlorethyl, Trichlormethyl, Trifluormethyl, 1,1-Dimethyl-2-chlorethyl und 2,2,2-Trifluorethyl,
gegebenenfalls durch ein oder mehrere Sauerstoff- und/oder Schwefelatome und/oder ein oder mehrere substituierte oder unsubstituierte Iminogruppen unterbrochenes C₆ - C₁₂-Aryl beispielsweise Phenyl, Tolyl, Xylyl, α-Naphthyl, β-Naphthyl, 4-Diphenylyl, Chlorphenyl, Dichlorphenyl, Trichlorphenyl, Difluorphenyl, Methylphenyl, Dimethylphenyl, Trimethylphenyl, Ethylphenyl, Diethylphenyl, *iso*-Propylphenyl, tert.-Butylphenyl, Dodecylphenyl, Methoxyphenyl, Dimethoxyphenyl, Ethoxyphenyl, Hexyloxyphenyl, Methylnaphthyl, Isopropylnaphthyl, Chlornaphthyl, Ethoxynaphthyl, 2,6-Dimethylphenyl, 2,4,6-Trimethylphenyl, 2,6-Dimethoxyphenyl, 2,6-Dichlorphenyl, 4-Bromphenyl, 2- oder 4-Nitrophenyl, 2,4- oder 2,6-Dinitrophenyl, 4-Dimethylaminophenyl, 4-Acetylphenyl, Methoxyethylphenyl oder Ethoxymethylphenyl, und bevorzugt Phenyl, Tolyl, Xylyl, α-Naphthyl, β-Naphthyl, 4-Diphenylyl, Chlorphenyl, Dichlorphenyl, Trichlorphenyl, Difluorphenyl, Methylphenyl, Dimethylphenyl, Trimethylphenyl, Ethylphenyl, Diethylphenyl, *iso*-Propylphenyl, tert.-Butylphenyl, Dodecylphenyl, Chlornaphthyl, 2,6-Dimethylphenyl, 2,4,6-Trimethylphenyl, 2,6-Dimethoxyphenyl, 2,6-Dichlorphenyl, 4-Bromphenyl, 2- oder 4-Nitrophenyl, 2,4- oder 2,6-Dinitrophenyl,
gegebenenfalls durch ein oder mehrere Sauerstoff- und/oder Schwefelatome und/oder ein oder mehrere substituierte oder unsubstituierte Iminogruppen unterbrochenes C₅ - C₁₂-Cycloalkyl beispielsweise Cyclopentyl, Cyclohexyl, Cyclooctyl, Cyclododecyl, Methylcyclopentyl, Dimethylcyclopentyl, Methylcyclohexyl, Dimethylcyclohexyl, Diethylcyclohexyl, Butylcyclohexyl, Methoxycyclohexyl, Dimethoxycyclohexyl, Diethoxycyclohexyl, Butylthiocyclohexyl, Chlorcyclohexyl, Dichlorcyclohexyl, Dichlorcyclopentyl sowie ein gesättigtes oder ungesättigtes bicyclisches System wie z.B. Norbornyl oder Norbornenyl, und bevorzugt Cyclopentyl, Cyclohexyl, Cyclooctyl, Cyclododecyl, Methylcyclopentyl, Dimethylcyclopentyl, Methylcyclohexyl, Dimethylcyclohexyl, Diethylcyclohexyl, Butylcyclohexyl, Chlorcyclohexyl, Dichlorcyclohexyl, Dichlorcyclopentyl sowie ein gesättigtes oder ungesättigtes bicyclisches System wie z.B. Norbornyl oder Norbornenyl
und
gegebenenfalls durch ein oder mehrere Sauerstoff- und/oder Schwefelatome und/oder ein oder mehrere substituierte oder unsubstituierte Iminogruppen unterbrochenes fünfbis sechsgliedrigen, Sauerstoff-, Stickstoff- und/oder Schwefelatome aufweisenden Heterocyclus beispielsweise Furyl, Thiophenyl, Pyrryl, Pyridyl, Indolyl, Benzoxazolyl, Dioxolyl, Dioxyl, Benzimidazolyl, Benzthiazolyl, Dimethylpyridyl, Methylchinolyl, Dimethylpyrryl, Methoxyfuryl, Dimethoxypyridyl, Difluorpyridyl, Methylthiophenyl, IsopropyIthiophenyl oder tert.-Butylthiophenyl.

Beispiele für R² sind eine Einfachbindung, Methylen, 1,1-Ethylen, 1,2-Ethylen, 1,1-Propylen, 1,2-Propylen, 1,3-Propylen, 1,1-Dimethyl-1,2-ethylen, 1,4-Butylen, 1,6-Hexylen, 2-Methyl-1,3-Propylen, 2-Ethyl-1,3-Propylen, 2,2-Dimethyl-1,3-Propylen und 2,2-Dimethyl-1,4-butylen, 3-Methyl-1,5-pentylen, 3,5-Heptylen, 1,2-Cyclopentylen, 1,3-Cyclopentylen, 1,2-Cyclohexylen,1,3-Cyclohexylen und ortho-Phenylen bevorzugt sind eine Einfachbindung, Methylen, 1,1-Ethylen, 1,2-Ethylen, 1,1-Propylen, 1,2-Propylen, 1,3-Propylen, 1,1-Dimethyl-1,2-ethylen und 3,5-Heptylen, besonders bevorzugt sind eine Einfachbindung, Methylen, 1,1-Ethylen, 1,2-Ethylen, 1,1-Propylen, 1,3-Propylen und 3,5-Heptylen, und ganz besonders bevorzugt sind 1,1-Propylen und 3,5-Heptylen.

Erfindungsgemäß sind R¹ und R³ bis R¹⁰ unabhängig voneinander Wasserstoff, C₁-C₄-Alkyl, n-Hexyl, n-Heptyl, n-Octyl, n-Decyl, n-Dodecyl, n-Tetradecyl, n-Hexadecyl, n-Octadecyl, n-Eicosyl, 2-Ethylhexyl, Cyclopentyl, Cyclohexyl, Cyclooctyl, Cyclododecyl, Phenyl, Naphthyl oder Benzyl.

C₁-C₄-Alkyl bedeutet im Rahmen dieser Schrift Methyl, Ethyl, *iso*-Propyl, n-Propyl, n-Butyl, *iso*-Butyl, *sek*-Butyl oder *tert-*Butyl*.* Erfindungsgemäß bedeutet C₁-C₄-Alkyl Methyl, Ethyl, n-Propyl und n-Butyl, besonders bevorzugt Methyl, Ethyl und n-Butyl und ganz besonders bevorzugt Methyl und Ethyl.
Bevorzugte Beispiele für Polyalkohole (C) sind 2-Ethyl-1,3-hexandiol, 2-Methyl-1,3-pentandiol, 2-Propyl-1,3-heptandiol, 2,4-Diethyloctan-1,5-diol, 2,2-Dimethyl-1-phenyl-1,3-propandiol, 2-Methyl-1,5-pentandiol, 3-Methyl-2,6-heptandiolund 4-Methyl-cyclohexan-1,3-diol. Offenbart ist auch 3,3-Dimethyl-1,2-butaniol. Besonders bevorzugt sind 2-Ethyl-1,3-hexandiol, 2-Methyl-1,3-pentandiol und 2,4-Diethyloctan-1,5-diol und ganz besonders bevorzugt sind 2-Ethyl-1,3-hexandiol und 2,4-Diethyloctan-1,5-diol.

Zur Unterscheidbarkeit der Hydroxygruppen ist es erfindungsgemäß erforderlich, daß die Hydroxygruppen eine unterschiedliche Umgebung aufweisen, also beispielsweise keine Spiegelebene (σ) aufweisen, wie es beispielsweise bei 2-Methyl-1,3-Propandiol der Fall ist und/oder keine C₂- und/oder C₃-Drehachse aufweisen, wie beispielsweise bei 2,2-Dimethyl-1,3-Propandiol oder Trimethylolethan.

Im Reaktionsschritt erfolgt die Veresterung mit (Meth)acrylsäure oder bevorzugt die Umesterung des Polyalkohols (C) mit mindestens einem, bevorzugt einem (Meth)acrylat (D) in Anwesenheit mindestens eines, bevorzugt eines die Umesterung katalysierenden Enzyms (E).

Verbindungen (D) können (Meth)acrylsäure oder Ester von (Meth)acrylsäure mit einem gesättigten Alkohol sein, bevorzugt gesättigte C₁ - C₁₀-Alkylester oder C₃ - C₁₂-Cycloalkylester der (Meth)acrylsäure, besonders bevorzugt gesättigte C₁ - C₄-Alkylester der (Meth)acrylsäure.

Gesättigt bedeutet im Rahmen dieser Schrift Verbindungen ohne C-C-Mehrfachbindungen (außer selbstverständlich die C=C-Doppelbindung in den (Meth)acryleinheiten).

Beispiele für Verbindungen (D) sind (Meth)acrylsäuremethyl-, -ethyl-, -n-butyl-, butyl-, n-octyl- und -2-Ethylhexylester, 1,2-Ethylenglycoldi- und -mono(meth)acrylat, 1,4-Butandioldi- und -mono(meth)acrylat, 1,6-Hexandioldi- und -mono(meth)acrylat, Trimethylolpropantri(meth)acrylat und Pentaerythrittetra(meth)acrylat.

Besonders bevorzugt sind (Meth)acrylsäuremethyl-, -ethyl-, -n-butyl- und -2-Ethylhexylester und ganz besonders bevorzugt (Meth)acrylsäuremethyl-, -ethyl- und -n-butylester.

Soweit die genannten Alkohole optisch aktiv sind werden sie bevorzugt racemisch oder als Diastereomerengemische eingesetzt, es ist jedoch auch möglich, sie als reine Enantiomere bzw. Diastereomere oder als Enantiomerengemische einzusetzen.

Die enzymatische Ver- oder Umesterung mit einem (Meth)acrylat erfolgt im Allgemeinen bei 0 bis 100°C, bevorzugt 20 bis 80 °C, besonders bevorzugt 20 bis 70°C, ganz besonders bevorzugt 20 bis 60 °C.

Erfindungsgemäß einsetzbare Enzyme (E) sind beispielsweise ausgewählt unter Hydrolasen (E.C. 3.-.-.-), und unter diesen besonders unter den Esterasen (E.C. 3.1.-.-), Lipasen (E.C. 3.1.1.3), Glykosylasen (E.C. 3.2.-.-) und Proteasen (E.C. 3.4.-.-) in freier oder auf einem Träger chemisch oder physikalisch immobilisierter Form, bevorzugt Lipasen, Esterasen oder Proteasen und besonders bevorzugt Esterasen (E.C. 3.1.-.-). Ganz besonders bevorzugt sind Novozyme 435 (Lipase aus *Candida antarctica* B) o-der Lipase aus Alcaligenes sp., Aspergillus sp., Mucor sp., Penicilium sp., Geotricum sp., Rhizopus sp., Burkholderia sp., Candida sp., Pseudomonas sp., Thermomyces sp. oder Schweinepankreas, insbesondere bevorzugt sind Lipase aus *Candida antarctica* B oder aus Burkholderia sp.

Der Enzymgehalt im Reaktionsmedium liegt in der Regel im Bereich von etwa 0,1 bis 10 Gew.-%, bezogen auf den eingesetzten Alkohol (C).

Die Reaktionszeit hängt unter anderem von der Temperatur, der verwendeten Menge und der Aktivität des Enzymkatalysators und vom geforderten Umsatz ab sowie vom teilveresterten Alkohol. Bevorzugt wird die Reaktionszeit so angepaßt, daß der Umsatz der umzusetzenden im Alkohol (C) enthaltenden, d.h. der niedrigersubstituierten Hydroxyfunktionen mindestens 70%, bevorzugt mindestens 80, besonders bevorzugt mindestens 90, ganz besonders bevorzugt mindestens 95 %, insbesondere mindestens 97 % und speziell mindestens 98 % beträgt. In der Regel sind dafür 1 bis 72 Stunden, bevorzugt 3 bis 36 und besonders bevorzugt 3 bis 24 Stunden ausreichend.

Das molare Verhältnis von (Meth)acrylsäureverbindung (D) (bezogen auf die (Meth)acryleinheiten) zu teilveresterten Alkohol (C) (bezogen auf Hydroxygruppen) kann in einem weiten Bereich, wie z.B. im Verhältnis 100:1 bis 1:1, bevorzugt 50:1 bis 1:1, besonders bevorzugt 20:1 bis 1:1 und ganz besonders bevorzugt 10:1 bis 1:1, eingestellt werden.

Die Reaktion kann in organischen Lösungsmitteln oder deren Gemischen oder ohne Zusatz von Lösungsmitteln ablaufen. Bevorzugt wird kein Lösungsmittel zugesetzt. Die Ansätze sind in der Regel weitgehend wasserfrei (d.h. unter 10, bevorzugt unter 5, besonders bevorzugt unter 1 und ganz besonders bevorzugt unter 0,5 Vol% Wasserzusatz).

Geeignete organische Lösungsmittel sind solche für diese Zwecke bekannten, beispielsweise tertiäre Monoole, wie C₃-C₆-Alkohole, bevorzugt tert-Butanol, tert-Amylalkohol, Pyridin, Poly-C₁-C₄-alkylenglykoldi-C₁-C₄-alkylether, bevorzugt Polyethyl-englycoldi-C₁-C₄-alkylether, wie z.B. 1,2-Dimethoxyethan, Diethylenglycoldimethylether, Polyethylenglycoldimethylether 500, Methyl-tert.Butylether, Ethyl-tert.Butylether, C₁-C₄-Alkylencarbonate, insbesondere Propylencarbonat, C₃-C₆-Alkylessigsäureester, insbesondere tert.-Butyl-essigsäureester, THF, Toluol, 1,3-Dioxolan, Aceton, *iso*-Butylmethylketon, Ethylmethylketon, 1,4-Dioxan, tert-Butylmethylether, Cyclohexan, Methylcyclohexan, Toluol, Hexan, Dimethoxymethan, 1,1-Dimethoxyethan, Acetonitril, sowie deren ein- oder mehrphasige Mischungen. Es kann vorteilhaft sein, freiwerdendes Wasser oder Alkohol durch ein möglichst nahe am Temperaturoptimum des verwendeten Enzyms siedendes binäres oder ternäres Heteroazeotrop abzutrennen. Der so entfernte Alkohol kann dann durch Phasenscheidung oder Membrandampftrennung entfernt werden.

Wahlweise können zu den organischen Lösungsmitteln wässrige Lösungsmittel zugesetzt werden, so dass - je nach organischem Lösungsmittel - ein- oder mehrphasige Reaktionslösungen entstehen. Beispiele für wässrige Lösungsmittel sind Wasser sowie wässrige, verdünnte (z.B. 10 bis 100mM) Puffer, beispielsweise mit einem pH-Wert im Bereich von etwa 6 bis 8, wie z.B. Kaliumphosphat- oder TRIS-HCI-Puffer.

Der Wasseranteil im Reaktionsansatz liegt erfindungsgemäß bei 0-10 Vol%. Bevorzugt werden die Reaktanden ohne Vorbehandlung (Trocknung, Wasserdotierung) eingesetzt.

Die Substrate liegen entweder gelöst, als Feststoffe suspendiert oder in Emulsion im Reaktionsmedium vor. Vorzugsweise liegt die anfängliche Konzentration der Reaktanden im Bereich von etwa 0,1 bis 20 Mol/l insbesondere bei 0,15 bis 10 Mol/l oder 0,2 bis 5 mol/l liegt.

Die Reaktion kann kontinuierlich, beispielsweise in einem Rohrreaktor oder in einer Rührreaktorkaskade, oder diskontinuierlich erfolgen.

Die Umsetzung kann in allen für eine solche Umsetzung geeigneten Reaktoren durchgeführt werden. Solche Reaktoren sind dem Fachmann bekannt. Bevorzugt erfolgt die Umsetzung in einem Rührkesselreaktor oder einem Festbettreaktor.

Zur Durchmischung des Reaktionsansatzes können beliebige Verfahren eingesetzt werden. Spezielle Rührvorrichtungen sind nicht erforderlich. Das Reaktionsmedium kann ein- oder mehrphasig sein und die Reaktanden werden darin gelöst, suspendiert oder emulgiert, gegebenenfalls zusammen mit dem Molekularsieb vorgelegt und zum Start der Reaktion, sowie gegebenenfalls ein- oder mehrmals im Verlauf der Reaktion, mit dem Enzympräparat versetzt. Die Temperatur wird während der Reaktion auf den gewünschten Wert eingestellt und kann, falls gewünscht, während des Reaktionsverlauf erhöht oder verringert werden.

Wird die Reaktion im Festbettreaktor durchgeführt, so ist der Festbettreaktor bevorzugt mit immobilisierten Enzymen bestückt, wobei die Reaktionsmischung durch eine mit dem Enzym gefüllte Säule gepumpt wird. Es ist auch möglich, die Umsetzung im Wirbelbett durchzuführen, wobei das Enzym auf einem Träger immobilisiert eingesetzt wird. Die Reaktionsmischung kann kontinuierlich durch die Säule gepumpt werden, wobei mit der Fließgeschwindigkeit die Verweilzeit und damit der gewünschte Umsatz steuerbar ist. Es ist auch möglich, die Reaktionsmischung im Kreislauf durch eine Säule zu pumpen, wobei auch unter Vakuum der freigesetzte Alkohol gleichzeitig abdestilliert werden kann.

Die Entfernung von Wasser im Falle einer Veresterung oder Alkoholen, die bei einer Umesterung aus den Alkyl(meth)acrylaten freigesetzt werden, erfolgt kontinuierlich oder schrittweise in an sich bekannter Weise, z.B. durch Vakuum, azeotrope Entfernung, Absorption, Pervaporation und Diffusion über Membranen.

Hierzu eignen sich vorzugsweise Molekularsiebe oder Zeolithe (Porengröße z.B. im Bereich von etwa 3-10 Angström), eine Abtrennung durch Destillation oder mit Hilfe geeigneter semipermeabler Membranen.

Es ist aber auch möglich, das abgetrennte Gemisch aus Alkyl(meth)acrylat und dem diesem zugrundeliegenden Alkohol, das häufig ein Azeotrop bildet, direkt in eine Anlage zur Herstellung des Alkyl(meth)acrylats zuzuführen, um es dort in einer Veresterung mit (Meth)acrylsäure wiederzuverwerten.

Nach Beendigung der Reaktion kann man das aus der Ver- oder Umesterung erhaltene Reaktionsgemisch ohne weitere Aufreinigung weiterverwenden oder es erforderlichenfalls in einem weiteren Schritt aufreinigen.

In der Regel wird in einem Reinigungsschritt lediglich das eingesetzte Enzym vom Reaktionsgemisch abgetrennt und das Reaktionsprodukt vom gegebenenfalls verwendeten organischen Lösungsmittel abgetrennt.

Eine Abtrennung vom Enzym erfolgt in der Regel durch Filtration, Absorption, Zentrifugation oder Dekantieren. Das abgetrennte Enzym kann anschließend für weitere Reaktionen eingesetzt werden.

Die Abtrennung vom organischen Lösungsmittel erfolgt in der Regel durch Destillation, Rektifikation oder bei festen Reaktionsprodukten durch Filtration.

Zur weiteren Aufreinigung des Reaktionsproduktes kann auch eine Chromatographie durchgeführt werden.

Bevorzugt werden im Reinigungsschritt jedoch lediglich das eingesetzte Enzym und das gegebenenfalls eingesetzte Lösungsmittel abgetrennt.

Die Reaktionsbedingungen bei der enzymatischen Ver- oder Umesterung sind mild. Aufgrund der niedrigen Temperaturen und sonstigen milden Bedingungen wird die Bildung von Nebenprodukten während der Reaktion vermieden, die andernfalls zum Beispiel von chemischen Katalysatoren stammen können oder durch unerwünschte radikalische Polymerisation des eingesetzten (Meth)acrylats, die sonst nur durch Zugabe von Stabilisatoren verhindert werden kann.

Bei der erfindungsgemäßen Reaktionsführung kann der (Meth)acrylverbindung (D) über den ohnehin enthaltenen Lagerstabilisator hinaus zusätzliche Stabilisator zugegeben werden, beispielsweise Hydrochinonmonomethylether, Phenothiazin, Phenole, wie z.B. 2-tert.-Butyl-4-methylphenol, 6-tert.-Butyl-2,4-dimethyl-phenol oder N-Oxyle, wie 4-Hydroxy-2,2,6,6-tetramethyl-piperidin-N-oxyl, 4-Oxo-2,2,6,6-tetramethyl-piperidin-N-oxyl, beispielsweise in Mengen von 50 bis 2000 ppm. Vorteilhaft wird die Ver- oder Umesterung in Gegenwart eines sauerstoffhaltigen Gases, bevorzugt Luft oder Luft-Stickstoff-Gemische, durchgeführt.

Des weiteren kann der Enzymkatalysator unproblematisch vom Endprodukt entfernt werden.

Das Reaktionsgemisches kann gegebenenfalls falls gewünscht aufgereinigt werden, beispielsweise durch Filtration, Destillation, Rektifikation, Chromatographie, Behandlung mit Ionentauschern, Adsorbentien, neutraler, saurer und/oder alkalischer Wäsche, Strippen oder Kristallisation.

Hierin auch offenbart sind die aus den Polyalkoholen (C) mit unterschiedlichen Hydroxygruppen erhältlichen (Meth)acrylate, bei denen ausschließlich die niedrigersubstituierten Hydroxygruppen (meth)acryliert sind, nicht jedoch die höhersubstituierten Hydroxygruppen.

Bevorzugt sind dies die Verbindungen der Formel Ia 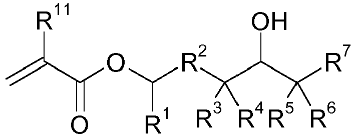 und Formel IIa, 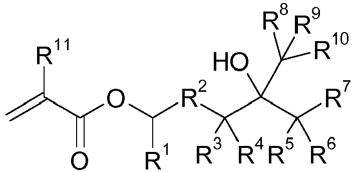 und Formel IIIa 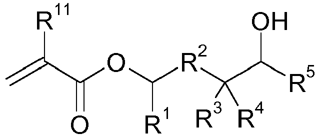 worin
R¹ bis R¹⁰ wie oben definiert sind und
R¹¹ Wasserstoff oder Methyl bedeutet.

Besonders bevorzugt sind (Meth)acrylsäure 2-Ethyl-3-hydroxy-hexyl ester, (Meth)acrylsäure 2-Methyl-3-hydroxy-pentyl ester und (Meth)acrylsäure 2,4-Diethyl-1,5-dihydroxy-octyl ester, ganz bevorzugt sind Acrylsäure 2-Ethyl-3-hydroxy-hexyl ester, Acrylsäure 2-Methyl-3-hydroxy-pentyl ester und Acrylsäure 2,4-Diethyl-1,5-dihydroxy-octyl ester, insbesondere Acrylsäure 2-Ethyl-3-hydroxy-hexyl ester und Acrylsäure 2,4-Diethyl-1,5-dihydroxy-octyl ester und speziell Acrylsäure 2,4-Diethyl-1,5-dihydroxy-octyl ester.

Die Farbzahl der erfindungsgemäß erhaltenen teilveresterten (Meth)Acrylsäureester beträgt in der Regel unter 100 APHA gemäß DIN ISO 6271, bevorzugt unter 80, besonders bevorzugt unter 60, ganz besonders bevorzugt unter 40 und insbesondere unter 20 APHA.

So erhältliche Beschichtungen weisen sehr hohe Kratzfestigkeiten, Härten, Chemikalienbeständigkeiten, Elastizität und Haftung, sowohl auf hydrophilen als auch auf hydrophoben Substraten auf.

Die erfindungsgemäß erhältlichen teilveresterten (Meth)acrylsäureester (F) können vorteilhaft als Monomere oder Comonomere in Poly(meth)acrylaten oder als Reaktivverdünner in thermisch-, strahlungs- und/oder Dual-Cure-härtbaren Poly(meth)acrylaten eingesetzt werden. Derartige Poly(meth)acrylate sind beispielsweise als Bindemittel in thermisch-, strahlungs- oder Dual-Cure-härtbaren Beschichtungsmitteln sowie in Klebstoffen, z.B. Acrylatklebstoffen geeignet sowie in Dichtungsmassen. Weiterhin sind die teilveresterten (Meth)acrylsäureester (F) verwendbar in Polyurethanen, beispielsweise PU-Dispersionen, PU-Schäumen, PU-Klebstoffen und PU-Beschichtungen. Unter thermisch härtbar versteht man z.B., 1 K- und 2K-Lacksysteme, die noch zusätzlich mit vernetzenden Reagenzien, z.B. Melaminharze oder Isocyanatderivate, umgesetzt werden.

Auch offenbart ist daher die Verwendung der nach dem erfindungsgemäßen Verfahren hergestellten teilveresterten (Meth)acrylsäureester als Reaktivverdünner oder Bindemittel in Strahlen- oder Dual-Cure-härtbaren Beschichtungsmassen, bevorzugt in Deckbeschichtungen, besonders bevorzugt in transparenten Klarlacken. Selbstverständlich können die erfindungsgemäß hergestellten teilveresterten (Meth)acrylsäureester auch als Monomere in Polymerisationen, gegebenenfalls zusammen mit anderen polymerisierbaren Monomeren, wie z.B. (Meth)acrylsäure, (Meth)acrylsäureester, Styrol, Butadien, Acrylnitril, Vinylacetat, N-Vinylpyrrolidon, 4-Hydroxybutylvinylether oder N-Vinylformamid, verwendet werden.

Unter "Dual-Cure" ist zu verstehen, dass die Beschichtungsmassen thermisch und mit aktinischer Strahlung härtbar sind. Wie hierin verwendet, ist unter aktinischer Strahlung elektromagnetische Strahlung wie sichtbares Licht, UV-Strahlung oder Röntgenstrahlung, insbesondere UV-Strahlung, und Korpuscularstrahlung wie Elektronenstrahlung zu verstehen.

Strahlenhärtbare Bindemittel sind solche, die mittels aktinischer Strahlung wie vorstehend definiert, insbesondere mittels UV-Strahlung härtbar sind.

Auch offenbart sind Lackformulierungen, enthaltend die nach dem erfindungsgemäßen Verfahren erhältlichen teilveresterten (Meth)acrylsäureester. Dabei können die teilveresterten (Meth)acrylsäureester sowohl in Basislacken als auch in Decklacken eingesetzt werden. Aufgrund ihrer besonderen Eigenschaften, wie der Erhöhung der Kratzfestigkeit und Elastizität, sowie der Erniedrigung der Viskosität, insbesondere bei verzweigten Polyacrylaten, einer strahlengehärteten Klarlackbeschichtung, ist ihr Einsatz in Deckbeschichtungen bevorzugt.

Neben den nach dem erfindungsgemäßen Verfahren erhältlichen teilveresterten (Meth)acrylsäureester (F) kann eine strahlungshärtbare Masse noch folgende Komponenten enthalten:
(G) mindestens eine polymerisierbare Verbindung mit mehreren copolymerisierbaren, ethylenisch ungesättigten Gruppen,
(H) gegebenenfalls Reaktivverdünner,
(I) gegebenenfalls Photoinitiator sowie
(J) gegebenenfalls weitere lacktypische Additive.

Als Verbindungen (G) kommen strahlungshärtbare, radikalisch polymerisierbare Verbindungen mit mehreren, d.h. mindestens zwei, copolymerisierbaren, ethylenisch ungesättigten Gruppen in Betracht.

Bevorzugt handelt es sich bei Verbindungen (G) um Vinylether- oder (Meth)acrylatverbindungen, besonders bevorzugt sind jeweils die Acrylatverbindungen, d.h. die Derivate der Acrylsäure.

Bevorzugte Vinylether- und (Meth)acrylat-Verbindungen (G) enthalten 2 bis 20, bevorzugt 2 bis 10 und ganz besonders bevorzugt 2 bis 6 copolymerisierbare, ethylenisch ungesättigte Doppelbindungen.

Besonders bevorzugt sind solche Verbindungen (G) mit einem Gehalt an ethylenisch ungesättigte Doppelbindungen von 0,1 - 0,7 mol /100 g, ganz besonders bevorzugt 0,2 -0,6mol/100g.

Das zahlenmittlere Molekulargewicht Mₙ der Verbindungen (G) liegt, wenn nicht anders angegeben, bevorzugt unter 15000, besonders bevorzugt bei 300 - 12000,
ganz besonders bevorzugt bei 400 bis 5000 und insbesondere bei 500 - 3000 g/mol (bestimmt durch Gelpermeationschromatographie mit Polystyrol als Standard und Tetrahydrofuran als Elutionsmittel).

Als (Meth)acrylatverbindungen genannt seien (Meth)acrylsäureester und insbesondere Acrylsäureester sowie Vinylether von mehrfunktionellen Alkoholen, insbesondere solchen, die neben den Hydroxylgruppen keine weiteren funktionellen Gruppen oder allenfalls Ethergruppen enthalten. Beispiele solcher Alkohole sind z.B. bifunktionelle Alkohole, wie Ethylenglykol, Propylenglykol und deren höher kondensierte Vertreter, z.B. wie Diethylenglykol, Triethylenglykol, Dipropylenglykol, Tripropylenglykol etc., 1,2-, 1,3-oder 1,4-Butandiol, 1,5-Pentandiol, 1,6-Hexandiol, 3-Methyl-1,5-pentandiol, Neopentylglykol, alkoxylierte phenolische Verbindungen, wie ethoxylierte bzw. propoxylierte Bisphenole, 1,2-, 1,3- oder 1,4-Cyclohexandimethanol, trifunktionelle und höherfunktionelle Alkohole, wie Glycerin, Trimethylolpropan, Butantriol, Trimethylolethan, Pentaerythrit, Ditrimethylolpropan, Dipentaerythrit, Sorbit, Mannit und die entsprechenden alkoxylierten, insbesondere ethoxylierten und/oder propoxylierten Alkohole.

Die Alkoxylierungsprodukte sind in bekannter Weise durch Umsetzung der vorstehenden Alkohole mit Alkylenoxiden, insbesondere Ethylen- oder Propylenoxid, erhältlich. Vorzugsweise beträgt der Alkoxylierungsgrad je Hydroxylgruppe 0 bis 10, d.h. 1 mol Hydroxylgruppe kann mit bis zu 10 mol Alkylenoxiden alkoxyliert sein.

Als (Meth)acrylatverbindungen seien weiterhin Polyester(meth)acrylate genannt, wobei es sich um die (Meth)acrylsäureester oder Vinylether von Polyesterolen handelt, sowie Urethan-, Epoxid- oder Melamin(meth)acrylate.

Urethan(meth)acrylate sind z.B. erhältlich durch Umsetzung von Polyisocyanaten mit Hydroxyalkyl(meth)acrylaten und gegebenenfalls Kettenverlängerungsmitteln wie Diolen, Polyolen, Diaminen, Polyaminen oder Dithiolen oder Polythiolen.

Die Urethan(meth)acrylate haben vorzugsweise ein zahlenmittleres Molgewicht Mₙ von 500 bis 20 000, insbesondere von 750 bis 10 000 besonders bevorzugt 750 bis 3000 g/mol (bestimmt durch Gelpermeationschromatographie mit Polystyrol als Standard).

Die Urethan(meth)acrylate haben vorzugsweise einen Gehalt von 1 bis 5, besonders bevorzugt von 2 bis 4 Mol (Meth)acrylgruppen pro 1000 g Urethan(meth)acrylat.

Epoxid(meth)acrylate sind erhältlich durch Umsetzung von Epoxiden mit (Meth)acrylsäure. Als Epoxide in Betracht kommen z.B epoxidierte Olefine oder Glycidylether, z.B. Bisphenol-A-diglycidylether oder aliphatische Glycidylether, wie Butandioldiglycidether.

Melamin(meth)acrylate sind erhältlich durch Umsetzung von Melamin mit (Meth)acrylsäure oder deren Ester.

Die Epoxid(meth)acrylate und Melamin(meth)acrylate haben vorzugsweise ein zahlenmittleres Molgewicht Mₙ von 500 bis 20000, besonders bevorzugt von 750 bis 10000 g/mol und ganz besonders bevorzugt von 750 bis 3000 g/mol; der Gehalt an (Meth)acrylgruppen beträgt vorzugsweise 1 bis 5, besonders bevorzugt 2 bis 4 pro 1000 g Epoxid(meth)acrylat oder Melamin(meth)acrylat (bestimmt durch Gelpermeationschromatographie mit Polystyrol als Standard und Tetrahydrofuran als Elutionsmittel).

Weiterhin geeignet sind Carbonat(meth)acrylate, die im Mittel vorzugsweise 1 bis 5, insbesondere 2 bis 4, besonders bevorzugt 2 bis 3 (Meth)acrylgruppen und ganz besonders bevorzugt 2(Meth)acrylgruppen enthalten.

Das zahlungsmittlere Molekulargewicht Mₙ der Carbonat(meth)acrylate ist vorzugsweise kleiner 3000 g/mol, besonders bevorzugt kleiner 1500 g/mol, besonders bevorzugt kleiner 800 g/mol (bestimmt durch Gelpermeationschromatgraphie mit Polystyrol als Standard, Lösemittel Tetrahydrofuran).

Die Carbonat(meth)acrylate sind in einfacher Weise erhältlich durch Umesterung von Kohlensäureestern mit mehrwertigen, vorzugsweise zweiwertigen Alkoholen (Diolen, z.B. Hexandiol) und anschließende Veresterung der freien OH-Gruppen mit (Meth)acrylsäure oder auch Umesterung mit (Meth)acrylsäureestern, wie es z.B. in EP-A 92 269 beschrieben ist. Erhältlich sind sie auch durch Umsetzung von Phosgen, Harnstoffderivaten mit mehrwertigen, z.B. zweiwertigen Alkoholen.

Als Reaktivverdünner (Verbindungen (H)) kommen strahlungshärtbare, radikalisch oder kationisch polymerisierbare Verbindungen mit nur einer ethylenisch ungesättigten, copolymerisierbaren Gruppe in Betracht.

Genannt seien z.B. C₁-C₂₀-Alkyl(meth)acrylate, Vinylaromaten mit bis zu 20 C-Atomen, Vinylester von bis zu 20 C-Atomen enthaltenden Carbonsäuren, ethylenisch ungesättigte Nitrile, Vinylether von 1 bis 10 C-Atome enthaltenden Alkoholen, α,β-ungesättigte Carbonsäuren und deren Anhydride und aliphatischen Kohlenwasserstoffen mit 2 bis 8 C-Atomen und 1 oder 2 Doppelbindungen.

Als (Meth)acrylsäurealkylester bevorzugt sind solche mit einem C₁-C₁₀-Alkylrest, wie Methylmethacrylat, Methylacrylat, n-Butylacrylat, Ethylacrylat und 2-Ethylhexylacrylat.

Insbesondere sind auch Mischungen der (Meth)acrylsäurealkylester geeignet.

Vinylester von Carbonsäuren mit 1 bis 20 C-Atomen sind z.B. Vinyllaurat, Vinylstearat, Vinylpropionat und Vinylacetat.

α,β-Ungesättigte Carbonsäuren und deren Anhydride können beispielsweise sein Acrylsäure, Methacrylsäure, Fumarsäure, Crotonsäure, Itaconsäure, Maleinsäure oder Maleinsäureanhydrid, bevorzugt Acrylsäure.

Als vinylaromatische Verbindungen kommen z.B. Vinyltoluol, α-Butylstyrol, 4-n-Butylstyrol, 4-n-Decylstyrol und vorzugsweise Styrol in Betracht.

Beispiele für Nitrile sind Acrylnitril und Methacrylnitril.

Geeignete Vinylether sind z.B. Vinylmethylether, Vinylisobutylether, Vinylhexylether und Vinyloctylether.

Als nicht aromatische Kohlenwasserstoffe mit 2 bis 8 C-Atomen und einer oder zwei olefinischen Doppelbindungen seien Butadien, Isopren, sowie Ethylen, Propylen und Isobutylen genannt.

Weiterhin sind N-Vinylformamid, N-Vinylpyrrolidon sowie N-Vinylcaprolactam einsetzbar.

Als Photoinitiatoren (I) können dem Fachmann bekannte Photoinitiatoren verwendet werden, z.B. solche in "Advances in Polymer Science", Volume 14, Springer Berlin 1974 oder in K. K. Dietliker, Chemistry and Technology of UV- and EB-Formulation for Coatings, Inks and Paints, Volume 3; Photoinitiators for Free Radical and Cationic Polymerization, P. K. T. Oldring (Eds), SITA Technology Ltd, London, genannten.

In Betracht kommen z.B. Mono- oder Bisacylphosphinoxide Irgacure 819 (Bis(2,4,6-Trimethylbenzoyl)phenylphosphinoxid), wie sie z.B. in EP-A 7 508, EP-A 57 474, DE-A 196 18 720, EP-A 495 751 oder EP-A 615 980 beschrieben sind, beispielsweise 2,4,6-Trimethylbenzoyldiphenylphosphinoxid (Lucirin^{®} TPO), Ethyl-2,4,6-trimethylbenzoylphenylphosphinat, Benzophenone, Hydroxyacetophenone, Phenylglyoxylsäure und ihre Derivate oder Gemische dieser Photoinitiatoren. Als Beispiele seien genannt Benzophenon, Acetophenon, Acetonaphthochinon, Methylethylketon, Valerophenon, Hexanophenon, α-Phenylbutyrophenon, p-Morpholinopropiophenon, Dibenzosuberon, 4-Morpholinobenzophenon, 4-Morpholinodeoxybenzoin, p-Diacetylbenzol, 4-Amino-benzophenon, 4'-Methoxyacetophenon, β-Methylanthrachinon, *tert*-Butylanthrachinon, Anthrachinoncarbonysäureester, Benzaldehyd, α-Tetralon, 9-Acetylphenanthren, 2-Acetylphenanthren, 10-Thioxanthenon, 3-Acetylphenanthren, 3-Acetylindol, 9-Fluorenon, 1-Indanon, 1,3,4-Triacetylbenzol, Thioxanthen-9-on, Xanthen-9-on, 2,4-Dimethylthioxanthon, 2,4-Diethylthioxanthon, 2,4-Di-*iso*-propylthioxanthon, 2,4-Dichlorthioxanthon, Benzoin, Benzoin-*iso*-butylether, Chloroxanthenon, Benzointetrahydropyranylether, Benzoin-methylether, Benzoin-ethylether, Benzoin-butylether, Benzoin-*iso*-propylether, 7-H-Benzoin-methylether, Benz[de]anthracen-7-on, 1-Naphthaldehyd, 4,4'-Bis(dimethylamino)benzophenon, 4-Phenylbenzophenon, 4-Chlorbenzophenon, Michlers Keton, 1-Acetonaphthon, 2-Acetonaphthon, 1-Benzoyl-cyclohexan-1-ol, 2-Hydroxy-2,2-dimethylacetophenon, 2,2-Dimethoxy-2-phenylace-tophenon, 2,2-Diethoxy-2-phenylacetophenon, 1,1-Dichloracetophenon, 1-Hydroxyacetophenon, Acetophenondimethylketal, o-Methoxybenzophenon, Triphenylphosphin, Tri-o-Tolylphosphin, Benz[a]anthracen-7,12-dion, 2,2-Diethoxy- acetophenon, Benzilketale, wie Benzildimethylketal, 2-Methyl-1-[4-(methylthio)phenyl]-2-morpholinopropan-1-on, Anthrachinone wie 2-Methylanthrachinon, 2-Ethylanthrachinon, 2-*tert*-Butylanthrachinon, 1-Chloranthrachinon, 2-Amylanthrachinon und 2,3-Butandion.

Geeignet sind auch nicht- oder wenig vergilbende Photoinitiatoren vom Phenylglyoxalsäureestertyp, wie in DE-A 198 26 712, DE-A 199 13 353 oder WO 98/33761 beschrieben.

Unter den genannten Photoinitiatoren sind Phosphinoxide, α-Hydroxyketone und Benzophenone bevorzugt.

Insbesondere können auch Gemische verschiedener Photoinitiatoren verwendet werden.

Die Photoinitiatoren können allein oder in Kombination mit einem Photopolymerisationspromotor, z.B. vom Benzoesäure-, Amin- oder ähnlichem Typ verwendet werden.

Als weitere lacktypische Additive (J) können beispielsweise Antioxidantien, Oxidationsinhibitoren, Stabilisatoren, Aktivatoren (Beschleuniger), Füllmittel, Pigmente, Farbstoffe, Entgasungsmittel, Glanzmittel, antistatische Agentien, Flammschutzmittel, Verdicker, thixotrope Agentien, Verlaufshilfsmittel, Bindemittel, Antischaummittel, Duftstoffe, oberflächenaktive Agentien, Viskositätsmodifikatoren, Weichmacher, Plastifizierer, klebrigmachende Harze (Tackifier), Chelatbildner oder Verträglichkeitsmittel (compatibilizer) verwendet werden.

Als Beschleuniger für die thermische Nachhärtung kann z.B. Zinnoctoat, Zinkoctoat, Dibutylzinnlaureat oder Diaza[2.2.2]bicyclooctan verwendet werden.

Weiterhin können ein oder mehrere photochemisch und/oder thermisch aktivierbare Initiatoren zugesetzt werden, z.B. Kaliumperoxodisulfat, Dibenzoylperoxid, Cyclohexanonperoxid, Di-tert.-Butylperoxid, Azobis-*iso*-butyronitril, Cyclohexylsulfonylacetylperoxid, Di-*iso*-propylpercarbonat, *tert*-Butylperoktoat oder Benzpinakol, sowie beispielsweise solche thermisch aktivierbare Initiatoren, die eine Halbwertszeit bei 80°C von mehr als 100 Stunden aufweisen, wie Di-t-Butylperoxid, Cumolhydroperoxid, Dicumyl-peroxid, t-Butylperbenzoat, silylierte Pinakole, die z. B. unter dem Handelsnamen AD-DID 600 der Firma Wacker kommerziell erhältlich sind oder Hydroxylgruppen-haltige Amin-N-Oxide, wie 2,2,6,6-Tetramethylpiperidin-N-oxyl, 4-Hydroxy-2,2,6,6-Tetra- methylpiperidin-N-oxyl etc.

Weitere Beispiele geeigneter Initiatoren sind in "Polymer Handbook", 2. Aufl., Wiley & Sons, New York beschrieben.

Als Verdicker kommen neben radikalisch (co)polymerisierten (Co)Polymerisaten, übliche organische und anorganische Verdicker wie Hydroxymethylcellulose oder Bentonite in Betracht.

Als Chelatbildner können z.B. Ethylendiaminessigsäure und deren Salze sowie β-Diketone verwendet werden.

Geeignete Füllstoffe umfassen Silikate, z. B. durch Hydrolyse von Siliciumtetrachlorid erhältliche Silikate wie Aerosil^{®} der Fa. Degussa, Kieselerde, Talkum, Aluminiumsilikate, Magnesiumsilikate, Calciumcarbonate etc.

Geeignete Stabilisatoren umfassen typische UV-Absorber wie Oxanilide, Triazine und Benzotriazol (letztere erhältlich als Tinuvin^{®} -Marken der Ciba-Spezialitätenchemie) und Benzophenone. Diese können allein oder zusammen mit geeigneten Radikalfängern, beispielsweise sterisch gehinderten Aminen wie 2,2,6,6-Tetramethylpiperidin, 2,6-Di-tert.-butylpiperidin oder deren Derivaten, z. B. Bis-(2,2,6,6-tetra-methyl-4-piperidyl)sebacinat, eingesetzt werden. Stabilisatoren werden üblicherweise in Mengen von 0,1 bis 5,0 Gew.-%, bezogen auf die in der Zubereitung enthaltenen festen Komponenten, eingesetzt.

Weiterhin geeignete Stabilisatoren sind beispielsweise N-Oxyle, wie z.B. 4-Hydroxy-2,2,6,6-tetramethyl-piperidin-N-oxyl, 4-Oxo-2,2,6,6-tetramethyl-piperidin-N-oxyl, 4-Acetoxy-2,2,6,6-tetramethyl-piperidin-N-oxyl, 2,2,6,6-tetramethyl-piperidin-N-oxyl, 4,4',4"-Tris(2,2,6,6-tetramethyl-piperidin-N-oxyl)-phosphit oder 3-Oxo-2,2,5,5-tetramethyl-pyrrolidin-N-oxyl, Phenole und Naphthole, wie z.B. p-Aminophenol, p-Nitrosophenol, 2-*tert*.-Butylphenol, 4-*tert*.-Butylphenol, 2,4-di-*tert*.-Butylphenol, 2-Methyl-4-*tert*.-Butylphenol, 4-Methyl-2,6-*tert*.-Butylphenol (2,6-*tert*.-Butyl-p-Kresol) oder 4-*tert*.-Butyl-2,6-dimethylphenol, Chinone, wie z.B. Hydrochinon oder Hydrochinonmonomethylether, aromatische Amine, wie z.B. N,N-Diphenylamin, N-Nitrosodiphenylamin, Phenylendiamine, wie z.B. N,N'-Dialkyl-para-phenylendiamin, wobei die Alkylreste gleich oder verschieden sein können und jeweils unabhängig voneinander aus 1 bis 4 Kohlenstoffatome bestehen und geradkettig oder verzweigt sein können, Hydroxylamine, wie z.B. N,N-Diethylhydroxylamin, Harnstoffderivate, wie z.B. Harnstoff oder Thioharnstoff, phosphorhaltige Verbindungen, wie z.B. Triphenylphosphin, Triphenylphosphit oder Triethylphosphit oder schwefelhaltige Verbindungen, wie z.B. Diphenylsulfid oder Phenothiazin.

Typische Zusammensetzungen für strahlungshärtbare Massen sind beispielsweise
(F) 20 - 100 Gew%, bevorzugt 40 - 90, besonders bevorzugt 50 - 90 und insbesondere 60 - 80 Gew%,
(G) 0 - 60 Gew%, bevorzugt 5 - 50, besonders bevorzugt 10 - 40 und insbesondere 10 - 30 Gew%,
(H) 0 - 50 Gew%, bevorzugt 5 - 40, besonders bevorzugt 6 - 30 und insbesondere 10 - 30 Gew%,
(I) 0 - 20 Gew%, bevorzugt 0,5 - 15, besonders bevorzugt 1 - 10 und insbesondere 2 - 5 Gew% sowie
(J) 0 - 50 Gew%, bevorzugt 2 - 40, besonders bevorzugt 3 - 30 und insbesondere 5 - 20 Gew%,
mit der Maßgabe, daß (F), (G), (H), (I) und (J) zusammen 100 Gew% ergeben.

Die Beschichtung der Substrate erfolgt nach üblichen, dem Fachmann bekannten Verfahren, wobei man wenigstens eine Beschichtungsmasse auf das zu beschichtende Substrat in der gewünschten Stärke aufbringt und die gegebenenfalls enthaltenen flüchtigen Bestandteile der Bechichtungsmasse, gegebenenfalls unter Erhitzen, entfernt. Dieser Vorgang kann gewünschtenfalls ein- oder mehrfach wiederholt werden. Das Aufbringen auf das Substrat kann in bekannter Weise, z. B. durch Spritzen, Spachteln, Rakeln, Bürsten, Rollen, Walzen, Gießen, Laminieren, Hinterspritzen oder Coextrudieren erfolgen. Die Beschichtungsstärke liegt in der Regel in einem Bereich von etwa 3 bis 1000 g/m² und vorzugsweise 10 bis 200 g/m².

Weiterhin wird ein Verfahren zum Beschichten von Substraten offenbart, bei dem man die Beschichtungsmasse auf das Substrat aufbringt und gegebenenfalls trocknet, mit Elektronenstrahlen oder UV Belichtung unter sauerstoffhaltiger Atmosphäre oder bevorzugt unter Inertgas härtet, gegebenenfalls bei Temperaturen bis zur Höhe der Trocknungstemperatur und anschließend bei Temperaturen bis zu 160°C, bevorzugt zwischen 60 und 160 °C, thermisch behandelt.

Das Verfahren zum Beschichten von Substraten kann auch so durchgeführt werden, daß nach dem Aufbringen der Beschichtungsmasse zunächst bei Temperaturen bis zu 160°C, bevorzugt zwischen 60 und 160 °C, thermisch behandelt und anschließend mit Elektronenstrahlen oder UV Belichtung unter Sauerstoff oder bevorzugt unter Inertgas gehärtet wird.

Die Härtung der auf dem Substrat gebildeten Filme kann gewünschtenfalls ausschließlich thermisch erfolgen. Im Allgemeinen härtet man die Beschichtungen jedoch sowohl durch Bestrahlung mit energiereicher Strahlung als auch thermisch.

Die Härtung kann auch zusätzlich oder anstelle der thermischen Härtung durch NIR-Strahlung erfolgen, wobei als NIR-Strahlung hier elektromagnetische Strahlung im Wel-lenlängenbereich von 760 nm bis 2,5 µm, bevorzugt von 900 bis 1500 nm bezeichnet ist.

Gegebenenfalls kann, wenn mehrere Schichten des Beschichtungsmittels übereinander aufgetragen werden, nach jedem Beschichtungsvorgang eine thermische, NIR und/oder Strahlungshärtung erfolgen.

Als Strahlungsquellen für die Strahlungshärtung geeignet sind z.B. Quecksilber-Niederdruckstrahler, -Mitteldruckstrahler mit Hochdruckstrahler sowie Leuchtstoffröhren, Impulsstrahler, Metallhalogenidstrahler, Elektronenblitzeinrichtungen, wodurch eine Strahlungshärtung ohne Photoinitiator möglich ist, oder Excimerstrahler. Die Strahlungshärtung erfolgt durch Einwirkung energiereicher Strahlung, also UV-Strahlung oder Tageslicht, vorzugsweise Licht im Wellenlängenbereich von λ=200 bis 700 nm strahlt, besonders bevorzugt von λ=200 bis 500 nm und ganz besonders bevorzugt λ=250 bis 400 nm, oder durch Bestrahlung mit energiereichen Elektronen (Elektronenstrahlung; 150 bis 300 keV). Als Strahlungsquellen dienen beispielsweise Hochdruckquecksilberdampflampen, Laser, gepulste Lampen (Blitzlicht), Halogenlampen oder Excimerstrahler. Die üblicherweise zur Vernetzung ausreichende Strahlungsdosis bei UV-Härtung liegt im Bereich von 80 bis 3000 mJ/cm².

Selbstverständlich sind auch mehrere Strahlungsquellen für die Härtung einsetzbar, z.B. zwei bis vier.
Diese können auch in jeweils unterschiedlichen Wellenlängebereichen strahlen.

Die Bestrahlung kann gegebenenfalls auch unter Ausschluß von Sauerstoff, z. B. unter Inertgas-Atmosphäre, durchgeführt werden. Als Inertgase eignen sich vorzugsweise Stickstoff, Edelgase, Kohlendioxid, oder Verbrennungsgase. Desweiteren kann die Bestrahlung erfolgen, indem die Beschichtungsmasse mit transparenten Medien abgedeckt wird. Transparente Medien sind z. B. Kunststofffolien, Glas oder Flüssigkeiten, z. B. Wasser. Besonders bevorzugt ist eine Bestrahlung in der Weise, wie sie in der DE-A1 199 57 900 beschrieben ist.

Auch offenbart ist ein Verfahren zur Beschichtung von Substraten, wobei man
i) ein Substrat mit einer Beschichtungsmasse, wie zuvor beschrieben, beschichtet,
ii) flüchtige Bestandteile der Beschichtungsmasse zur Filmbildung unter Bedingungen entfernt, bei denen der Photoinitiator (I) im wesentlichen noch keine freien Radikale ausbildet,
iii) gegebenenfalls den in Schritt ii) gebildeten Film mit energiereicher Strahlung bestrahlt, wobei der Film vorgehärtet wird, und anschließend gegebenenfalls den mit dem vorgehärteten Film beschichteten Gegenstand mechanisch bearbeitet oder die Oberfläche des vorgehärteten Films mit einem anderen Substrat in Kontakt bringt,
iv) dem Film thermisch oder mit NIR-Strahlung endhärtet

Dabei können die Schritte iv) und iii) auch in umgekehrter Reihenfolge durchgeführt, d. h. der Film kann zuerst thermisch oder per NIR-Strahlung und dann mit energiereicher Strahlung gehärtet werden.

Weiterhin sind auch Substrat, beschichtet mit einer Mehrschichtlackierung offenbart.

Die Dicke einer solche wie beschrieben zu härtenden Schicht kann von 0,1 µm bis mehrere mm betragen, bevorzugt von 1 bis 2000 µm, besonders bevorzugt 5 bis 1000 µm, ganz besonders bevorzugt von 10 bis 500 µm und insbesondere von 10 bis 250 µm.

Die erfindungsgemäß hergestellten teilveresterten (Meth)acrylsäureester können aufgrund ihrer geringeren Färbung vorteilhaft auch in einer thermisch induzierten (radikalischen) (Co)polymerisation eingesetzt werden.

Als Monomere, mit denen die erfindungsgemäß hergestellten teilveresterten (Meth)acrylsäureester beispielsweise copolymerisiert werden können seien genannt z.B. C₁-C₂₀-Alkyl(meth)acrylate, Vinylaromaten mit bis zu 20 C-Atomen, Vinylester von bis zu 20 C-Atomen enthaltenden Carbonsäuren, ethylenisch ungesättigte Nitrile, Vinylether von 1 bis 10 C-Atome enthaltenden Alkoholen und aliphatischen Kohlenwasserstoffen mit 2 bis 8 C-Atomen und 1 oder 2 Doppelbindungen.

Als (Meth)acrylsäurealkylester bevorzugt sind solche mit einem C₁-C₁₀-Alkylrest, wie Methylmethacrylat, Methylacrylat, n-Butylacrylat, Ethylacrylat und verzweigte Alkylderivate wie 2-Ethylhexylacrylat.

Insbesondere sind auch Mischungen der (Meth)acrylsäurealkylester geeignet.

Vinylester von Carbonsäuren mit 1 bis 20 C-Atomen sind z.B. Vinyllaurat, Vinylstearat, Vinylpropionat und Vinylacetat.

Als vinylaromatische Verbindungen kommen z.B. Vinyltoluol, α-Butylstyrol, 4-n-Butyl-styrol, 4-n-Decylstyrol und vorzugsweise Styrol in Betracht.

Beispiele für Nitrile sind Acrylnitril und Methacrylnitril.

Geeignete Vinylether sind z.B. Vinylmethylether, Vinylisobutylether, Vinylhexylether und Vinyloctylether.

Als nicht aromatische Kohlenwasserstoffe mit 2 bis 8 C-Atomen und einer oder zwei olefinischen Doppelbindungen seien Butadien, Isopren, sowie Ethylen, Propylen und Isobutylen genannt.

Eine häufige, aber nicht die einzige Methode zur Herstellung solcher (Co)Polymerisate ist die radikalische oder ionische (Co)Polymerisation in einem Lösungs- oder Verdünnungsmittel.

Die radikalische (Co)Polymerisation solcher Monomere erfolgt beispielsweise in wäßriger Lösung in Gegenwart von Polymerisationsinitiatoren, die unter Polymerisationsbedingungen in Radikale zerfallen, beispielsweise Peroxodisulfate, H₂O₂-Redoxysysteme oder Hydroxyperoxide, wie z.B. tert. Butylhydroperoxid oder Cumolhydroperoxid. Die (Co)Polymerisation kann in einem weiten Temperaturbereich, gegebenenfalls unter vermindertem oder auch unter erhöhtem Druck in der Regel bei Temperaturen bis zu 100 °C vorgenommen werden. Der pH-Wert des Reaktionsgemisches wird gewöhnlich in dem Bereich von 4 bis 10 eingestellt.

Die (Co)Polymerisation kann aber auch in anderer, dem Fachmann an sich bekannter Weise kontinuierlich oder diskontinuierlich durchgeführt werden, z.B. als Lösungs-, Fällungs-, Wasser-in-Öl-Emulsions-, inverse Emulsions, Suspensions oder umgekehrte Suspensionspolymerisation.

Dabei wird das Monomer/die Monomere unter Verwendung radikalischer Polymerisationsinitiatoren, z.B. in Radikale zerfallende Azoverbindungen, wie 2,2'-Azo-bis(isobutyronitril), 2,2'-Azobis-(2-amidinopropan)-hydrochlorid oder 4,4'-Azo-bis-(4'-cyan-pentansäure) oder Dialkylperoxide, wie Di-tert.-Amylperoxid, Aryl-alkylperoxide, wie tert.-Butyl-cumylperoxid, Alkyl-acylperoxide, wie tert.-Butyl-peroxy-2-ethylhexanoat, Peroxidicarbonate, wie Di-(4-tert.-Butylcyclohexyl)peroxydicarbonat oder Hydroperoxide (co)polymerisiert.

Die genannten Verbindungen werden meist in Form wäßriger Lösungen oder wäßriger Emulsionen eingesetzt, wobei die untere Konzentration durch die in der (Co)Polymerisation vertretbare Wassermenge und die obere Konzentration durch die Löslichkeit der betreffenden Verbindung in Wasser bestimmt ist.

Als Lösungs- oder Verdünnungsmittel können dienen z.B. Wasser, Alkohole, wie Methanol, Ethanol, n- oder iso-Propanol, n- oder iso-Butanol, oder Ketone, wie Aceton, Ethylmethylketon, Diethylketon oder *iso*-Butylmethylketon. Besonders bevorzugt sind unpolare Lösungsmittel wie beispielsweise Xylol und dessen Isomerengemische, Shellsol® A und Solventnaphtha.

Bevorzugt werden die Monomere vorgemischt und Initiator mit gegebenenfalls weiteren Zusätzen gelöst in Lösungsmittel zugegeben. Dies ist besonders bevorzugt beschrieben in WO 01/23484 und dort besonders auf Seite 10, Z. 3 bis Z. 24.

Gegebenenfalls kann die (Co)Polymerisation in Gegenwart von Polymerisationsreglern, wie beispielsweise Hydroxylammoniumsalze, chlorierte Kohlenwasserstoffe und Thioverbindungen, wie z.B. tert.-Butylmercaptan, Thioglycolsäureethylacrylester, Mercaptoethanol, Mercaptopropyltrimethoxysilan, Dodecylmercaptan, tert.-Dodecylmercaptan oder Alkalimetallhypophosphite, durchgeführt werden. Bei der (Co)Polymerisation können diese Regler, z. B. in Mengen von 0 bis 0,8 Gew.-Teile, bezogen auf 100 Gew.-Teile der zu (co)polymerisierenden Monomeren, eingesetzt werden, durch die die Molmasse des entstehenden (Co)Polymers verringert wird.

Bei der Emulsionspolymerisation können Dispergiermittel, ionische und/oder nichtionische Emulgatoren und/oder Schutzkolloide bzw. Stabilisatoren als grenzflächenaktive Verbindungen verwendet werden.

Als solche kommen sowohl die zur Durchführung von Emulsionspolymerisationen üblicherweise eingesetzten Schutzkolloide als auch Emulgatoren in Betracht.

Geeignete Schutzkolloide sind beispielsweise Polyvinylalkohole, Cellulosederivate oder Vinylpyrrolidon enthaltende Copolymerisate. Eine ausführliche Beschreibung weiterer geeigneter Schutzkolloide findet sich in Houben-Weyl, Methoden der organischen Chemie, Band XIV/1, makromolekulare Stoffe, Georg-Thieme-Verlag, Stuttgart, 1969, S. 411 bis 420. Selbstverständlich können auch Gemische aus Emulgatoren und/oder Schutzkolloiden verwendet werden. Vorzugsweise werden als Dispergiermittel ausschließlich Emulgatoren eingesetzt, deren relative Molekulargewichte im Unterschied zu den Schutzkolloiden üblicherweise unter 1000 liegen. Sie können sowohl anionischer, kationischer oder nichtionischer Natur sein. Selbstverständlich müssen im Falle der Verwendung von Gemischen granzflächenaktiver Substanzen die Einzelkomponenten miteinander verträglich sein, was im Zweifelsfall an Hand weniger Vorversuche überprüft werden kann. Im allgemeinen sind anionische Emulgatoren untereinander und mit nichtionischen Emulgatoren verträglich.

Desgleichen gilt auch für kationische Emulgatoren, während anionische und kationische Emulgatoren meistens miteinander unverträglich sind. Gebräuchliche Emulgatoren sind z.B. ethoxylierte Mono-, Di- und Tri-Alkylphenole (EO-Grad: 3 bis 100, C₄ bis C₁₂), ethoxylierte Fettalkohole (EO-Grad: 3 bis 100, Alkylrest: C₈ bis C₁₈), sowie Alkali- und Ammoniumsalze von Alkylsulfaten (Alkylrest: C₈ bis C₁₆) von Schwefelsäurehalbestern ethoxylierter Alkylphenole (EO-Grad: 3 bis 100, Alkylrest: C₄ bis C₁₂), von Alkylsulfonsäuren (Alkylrest: C₁₂ bis C₁₈) und von Alkylarylsulfonsäuren (Alkylrest: C₉ bis C₁₈). Weitere geeignete Emulgatoren wie Sulfobernsteinsäureester finden sich in Hou-ben-Weyl, Methoden der organischen Chemie, Band XIV/1, Makromolekulare Stoffe, Georg-Thieme Verlag, Stuttgart, 1961, Seiten 192 bis 208.

In der Regel beträgt die Menge an eingesetzten Dispergiermittel 0,5 bis 6, vorzugsweise 1 bis 3 Gew.-% bezogen auf die radikalisch zu polymerisierenden Monomeren.

Beispiele für (meth)acrylathaltige Dispersionen sind n-Butylacrylat/Acrylnitril-Dispersionen oder n-Butylacrylat/Butadien/Styrol-Dispersionen, die als Klebstoffe Anwendung finden.

Die Polymerdispersionen, in denen erfindungsgemäß hergestellte teilveresterten (Meth)acrylsäureester verwendet werden, können zusätzlich chemisch und/oder physikalisch desodoriert werden.

Die mit den erfindungsgemäß hergestellten teilveresterten (Meth)acrylsäureestern erhältlichen Copolymerisate weisen in der Regel eine geringere Farbzahl auf, was im Lackbereich vorteilhaft ist. Die beschriebenen Copolymerisate lassen sich dann in an sich bekannter Weise beispielsweise mit Aminoplasten, wie z.B. Melamin, zu vernetzten Lackharzen umsetzen, wie es beispielsweise beschrieben ist in der EP 738740 oder EP 675141.

Besonders bevorzugt eignen sich die offenbarten Beschichtungsmassen als oder in Außenbeschichtungen, also solche Anwendungen, die dem Tageslicht ausgesetzt sind, bevorzugt von Gebäuden oder Gebäudeteilen, Innenbeschichtungen, Straßenmarkierungen, Beschichtungen auf Fahrzeugen und Flugzeugen. Insbesondere werden die Beschichtungen als Holz-, Papier- oder Kunststoffbeschichtungen, beispielsweise für Parkett oder Möbel eingesetzt.

Mit Hilfe des erfindungsgemäßen Verfahrens ist die Herstellung teilveresterten (Meth)acrylsäureester (F) in hoher chemischer und Raum-Zeit-Ausbeute und unter milden Bedingungen mit guten Farbzahlen möglich. Trotz Verzicht auf Schutzgruppen und aktivierte (Meth)Acrylsäureverbindungen werden gezielt die gewünschten teilveresterten Produkte mit hoher Selektivität erhalten, die frei von Nebenprodukten sind. Liegen mehrer niedrigersubstituierte Hydroxygruppen vor, so werden in der Regel, bei ausreichender Menge an (Meth)acrylierungsreagens, alle niedrigersubstituierten Hydroxygruppen gleichmäßig substituiert, bevor höhersubstituierte Hydroxygruppen (meth)acryliert werden.

Die Selektivität der erfindungsgemäßen Reaktion bzgl. der (Meth)acrylierung der niedriger substituierten Hydroxygruppen gegenüber den niedriger substituierten Hydroxygruppen ist in der Regel mindestens 90:10, bevorzugt mindestens 95:5, besonders bevorzugt mindestens 97:3, ganz besonders bevorzugt mindestens 98:2 und insbesondere mindestens 99:1.

Der Anteil an mehrfach (meth)acryliertem Produkt beträgt nicht mehr als 5 mol% bezogen auf den Polyalkohol (C), bevorzugt nicht mehr als 3, besonders bevorzugt nicht mehr als 2 und insbesondere nicht mehr als 1 mol%.

Die folgenden Beispiele sollen die Eigenschaften der Erfindung erläutern, ohne sie aber einzuschränken.

### Beispiele

Als "Teile" seien in dieser Schrift, wenn nicht anders angegeben, "Gewichtsteile" verstanden.

### Beispiele 1 und 2

### DEOD-Monoacrylat (mit / ohne Lösungsmittel)

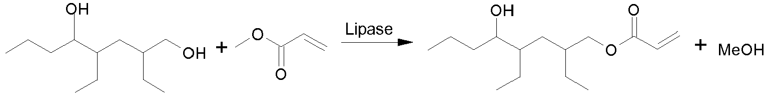

In einem Schraubdeckelglas wurden 5 mmol 2,4-Diethyloctan-1,5-diol (DEOD; 1,0 g) mit 50 mmol Methylacrylat (4,3 g), 50 mg Novozym® 435 (geträgerte Lipase, Fa. Novozymes Dänemark), evtl. 5,0 ml MTBE (tert-Butyl methylether) und 1,0 g Molekularsieb 5 Å für 24 h bei 40 °C geschüttelt.
Danach wurde von Feststoffen abfiltriert und eine Probe wurde silyliert und mittels GC analysiert. Das Diol war zu 96-98 % zum Monoacrylat umgesetzt worden.
Es wurde <0,5 % Diacrylat detektiert.

| Bedingungen | Umsatz [%] |
|---|---|
| ohne Lösungsmittel | 98 |
| MTBE | 96 |

### Beispiel 3

### DEOD-Monoacrylat (präparativ)

In einem Rundkolben wurden unter Rückfluß 2,5 mol 2,4-Diethyloctan-1,5-diol (DEOD; 506 g) mit 5,0 mol Methylacrylat (431 g), 25 g Novozym® 435 (geträgerte Lipase, Fa. Novozymes Dänemark) und 750 g Molekularsieb 5 Å für 23 h bei 60 °C gerührt. Danach wurde von Feststoffen abfiltriert, mit etwas MTBE nachgewaschen und Methylacrylat und MTBE am Rotationsverdampfer im Vakuum entfernt.

Man erhielt 406 g eines farblösen Flüssigkeit. Eine Probe wurde silyliert und mittels GC analysiert. Das isolierte Produkt enthielt demnach 0,3 % 2,4-Diethyloctan-1,5-diol und >99,5 % des am primären Alkohols acrylierten Produkts. Es wurde <0,5 % Diacrylat detektiert.

### Beispiel 4

### EHD-Monoacrylat

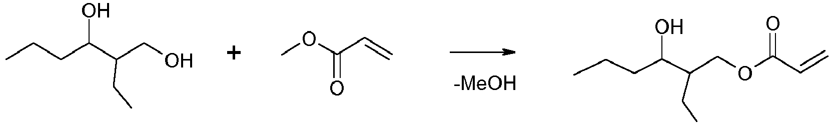

In einem Schraubdeckelglas wurden 5 mmol 2-Ethyl-1,3-hexandiol (EHD; 730 mg) mit 50 mmol Methylacrylat (4,3 g), 50 mg Novozym® 435 (geträgerte Lipase, Fa. Novozymes Dänemark), und 1,5 g Molekularsieb 5 Å für 8 h bei 60 °C geschüttelt. Danach wurde von Feststoffen abfiltriert, eine Probe silyliert und mittels GC analysiert. Das Diol war zu 98 % zum Monoacrylat umgesetzt worden. Es wurde <0,5 % Diacrylat detektiert.

### Beispiel 5

### DPPD-Monoacrylat

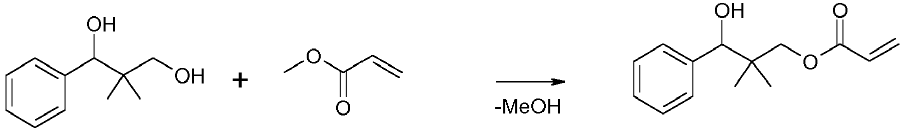

In einem Schraubdeckelglas wurden 5 mmol 2,2-Dimethyl-1-phenyl-1,3-propandiol (DPPD; 900 mg) mit 50 mmol Methylacrylat (4,3 g), 50 mg Novozym® 435 (geträgerte Lipase, Fa. Novozymes Dänemark), und 1,5 g Molekularsieb 5 Å für 8 h bei 40 °C geschüttelt. Danach wurde von Feststoffen abfiltriert, eine Probe silyliert und mittels GC analysiert. Das Diol war zu 99 % zum Monoacrylat umgesetzt worden. Es wurde <0,5 % Diacrylat detektiert.

### Beispiel 6 (nicht erfindungsgemäß)

### DBD-Monoacrylat

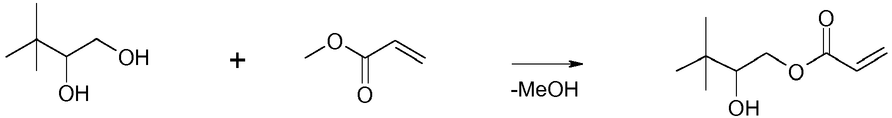

In einem Schraubdeckelglas wurden 5 mmol 3,3-Dimethyl-1,2-butandiol (DBD; 590 mg) mit 50 mmol Methylacrylat (4,3 g), 50 mg Novozym® 435 (geträgerte Lipase, Fa. Novozymes Dänemark), und 1,0 g Molekularsieb 5 Å für 24 h bei 40 °C geschüttelt. Danach wurde von Feststoffen abfiltriert, eine Probe silyliert und mittels GC analysiert. Das Diol war zu 97 % zum Monoacrylat umgesetzt worden. Es wurde <0,5 % Diacrylat detektiert.

## Patentansprüche

1. Verfahren zur Herstellung von teilveresterten (Meth)acrylsäureestern (F) von zweiwertigen Polyalkoholen (C), die unterschiedliche Hydroxygruppen enthalten, **dadurch gekennzeichnet, dass** man
entweder
(1) mindestens einen Polyalkohol (C₁) mit einer primären Hydroxygruppe und einer sekundären Hydroxygruppe,
oder
(2) mindestens einen Polyalkohol (C₂) mit einer primären Hydroxyguppe und einer tertiären Hydroxygruppe,
in Gegenwart mindestens eines Enzyms (E) mit (Meth)acrylsäure verestert oder mindestens einem (Meth)acrylsäureester (D) umestert, wobei der Anteil an mehrfach (meth)acryliertem Produkt nicht mehr als 5 mol%, bezogen auf den Polyalkohol (C), beträgt,
wobei der Wasseranteil im Reaktionsansatz bei 0-10 Vol% liegt, **dadurch gekennzeichnet, dass** es sich bei dem Polyalkohol (C) um einen der Formel II 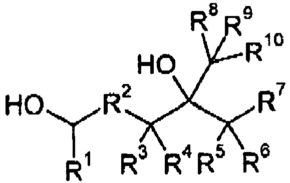 oder Formel III handelt 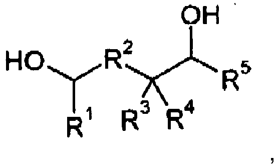 worin
R¹ und R³ bis R¹⁰ unabhängig voneinander ausgewählt sind aus der Gruppe umfassend Wasserstoff, Methyl, Ethyl, n-Propyl, n-Butyl, n-Hexyl, n-Heptyl, n-Octyl, n-Decyl, n-Dodecyl, n-Tetradecyl, n-Hexadecyl, n-Octadecyl, n-Eicosyl, 2-Ethylhexyl, Cyclopentyl, Cyclohexyl, Cyclooctyl, Cyclododecyl, Phenyl, Naphthyl oder Benzyl, und
R² eine Einfachbindung, C₁-C₂₀-Alkylen, C₅-C₁₂-Cycloalkylen, C₆-C₁₂-Arylen oder durch ein- oder mehrere Sauerstoff- und/oder Schwefelatome und/oder ein oder mehrere substituierte oder unsubstituierte Iminogruppen und/oder durch eine o-der mehrere Cycloalkyl-, -(CO)-, -O(CO)O-, -(NH)(CO)O-, -O(CO)(NH)-, -O(CO)-oder -(CO)O-Gruppen unterbrochenes C₂-C₂₀-Alkylen, wobei die genannten Reste jeweils durch Aryl, Alkyl, Aryloxy, Alkyloxy, Heteroatome und/oder Heterocyclen substituiert sein können,
bedeuten,
wobei
für den Fall (2) in Anspruch 1 in Formel II R¹ Wasserstoff ist und mindestens einer der Reste R³ bis R¹⁰ ungleich Wasserstoff ist.

2. Verfahren nach dem vorstehenden Anspruch, **dadurch gekennzeichnet, dass** R² ausgewählt ist aus der Gruppe umfassend ein Einfachbindung, Methylen, 1,1-Ethylen, 1,2-Ethylen, 1,1-Propylen, 1,2-Propylen, 1,3-Propylen, 1,1-Dimethyl-1,2-ethylen, 1,4-Butylen, 1,6-Hexylen, 2-Methyl-1,3-Propylen, 2-Ethyl-1,3-Propylen, 2,2-DimethyM ,3-Propylen und 2,2-Dimethyl-1,4-butylen, 3-Methyl-1,5-pentylen, 3,5-Heptylen, 1,2-Cyclopentylen, 1,3-Cyclopentylen, 1,2-Cyclohexylen,1,3-Cyclohexylen und ortho-Phenylen.

3. Verfahren nach einem der vorstehenden Ansprüche, **dadurch gekennzeichnet, dass** der Polyalkohol ausgewählt ist aus der Gruppe umfassend 2-Ethyl-1,3-hexandiol, 2-Methyl-1,3-pentandiol, 2-Propyl-1,3-heptandiol, 2,4-Diethyloctan-1,5-diol und 2,2-Dimethyl-1-phenyl-1,3-propandiol.

4. Verfahren nach einem der vorstehenden Ansprüche, **dadurch gekennzeichnet, dass** das Enzym (E) ausgewählt ist unter Esterasen (E.C. 3.1.-.-), Lipasen (E.C. 3.1.1.3), Glykosylasen (E.C. 3.2.-.-) und Proteasen (E.C. 3.4.-.-).

## Claims

1. A process for preparing partial (meth)acrylic esters (F) of dihydric polyalcohols (C) containing different hydroxyl groups, which comprises subjecting
alternatively
(1) at least one polyalcohol (C₁) having a primary hydroxyl group and a secondary hydroxyl group, or
(2) at least one polyalcohol (C₂) having a primary hydroxyl group and a tertiary hydroxyl group,
to esterification with (meth)acrylic acid or to transesterification with at least one (meth)acrylic ester (D) in the presence of at least one enzyme (E), where the proportion of poly(meth)acrylated product is not more than 5 mol%, based on the polyalcohol (C),
where the proportion of water in the reaction batch is 0-10" by volume, wherein the polyalcohol (C) is a polyalcohol of the formula II 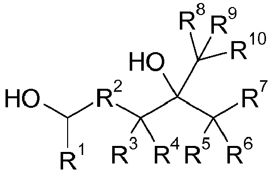 or formula III 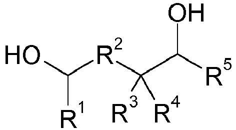 in which
R¹ and R³ to R¹⁰ independently of one another are selected from the group comprising hydrogen, methyl, ethyl, n-propyl, n-butyl, n-hexyl, n-heptyl, n-octyl, n-decyl, n-dodecyl, n-tetradecyl, n-hexadecyl, n-octadecyl, n-eicosyl, 2-ethylhexyl, cyclopentyl, cyclohexyl, cyclooctyl, cyclododecyl, phenyl, naphthyl and benzyl,and
R² is a single bond, C₁-C₂₀ alkylene, C₅-C₁₂ cycloalkylene, C₆-C₁₂ arylene or C₂-C₂₀ alkylene which is interrupted by one or more oxygen and/or sulfur atoms and/or one or more substituted or unsubstituted imino groups and/or one or more cycloalkyl, -(CO)-, -O(CO)O-, -(NH)(CO)O-, -O(CO)(NH)-, -O(CO)- or -(CO)O- groups, it being possible for the specified radicals to be substituted in each case by aryl, alkyl, aryloxy, alkyloxy, heteroatoms and/or heterocycles,
where
for the case (2) in claim 1 in formula II R¹ is hydrogen and at least one of the radicals R³ to R¹⁰ is other than hydrogen.

2. The process according to any one of the preceding claims, wherein R² is selected from the group comprising a single bond, methylene, 1,1-ethylene, 1,2-ethylene, 1,1-propylene, 1,2-propylene, 1,3-propylene, 1,1-dimethyl-1,2-ethylene, 1,4-butylene, 1,6-hexylene, 2-methyl-1,3-propylene, 2-ethyl-1,3-propylene, 2,2-dimethyl-1,3-propylene and 2,2-dimethyl-1,4-butylene, 3-methyl-1,5-pentylene, 3,5-heptylene, 1,2-cyclopentylene, 1,3-cyclopentylene, 1,2-cyclohexylene, 1,3-cyclohexylene and orthophenylene.

3. The process according to any one of the preceding claims, wherein the polyalcohol is selected from the group comprising 2-ethyl-1,3-hexanediol, 2-methyl-1,3-pentanediol, 2-propyl-1,3-heptanediol, 2,4-diethyloctane-1,5-diol and 2,2-dimethyl-1-phenyl-1,3-propanediol.

4. The process according to any one of the preceding claims, wherein the enzyme (E) is selected from esterases (E.C. 3.1.-.-), lipases (E.C. 3.1.1.3), glycosylases (E.C. 3.2.-.-) and proteases (E.C. 3.4.-.-).

## Revendications

1. Procédé pour la préparation d'esters de l'acide (méth)acrylique (F) partiellement estérifiés de polyalcools (C) divalents, qui contiennent des groupes hydroxy différents, **caractérisé en ce que** soit
(1) au moins un polyalcool (C₁) présentant un groupe hydroxy primaire et un groupe hydroxy secondaire, soit
(2) au moins un polyalcool (C₂) présentant un groupe hydroxy primaire et un groupe hydroxy tertiaire
est estérifié avec de l'acide (méth)acrylique ou transestérifié avec au moins un ester de l'acide (méth)acrylique (D) en présence d'au moins une enzyme (E), la proportion de produit poly(méth)acrylé n'étant pas supérieure à 5% en mole, par rapport au polyalcool C, la proportion d'eau dans la charge réactionnelle étant de 0-10% en volume, **caractérisé en ce qu'**il s'agit, pour le polyalcool (C), d'un polyalcool de formule II 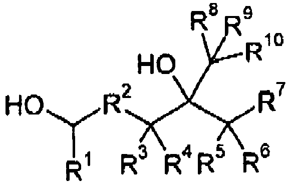 ou de formule III 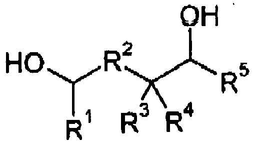 dans lesquelles
R¹ et R³ à R¹⁰ sont choisis, indépendamment les uns des autres, dans le groupe comprenant hydrogène, méthyle, éthyle, n-propyle, n-butyle, n-hexyle, n-heptyle, n-octyle, n-décyle, n-dodécyle, n-tétradécyle, n-hexadécyle, n-octadécyle, n-eicosyle, 2-éthylhexyle, cyclopentyle, cyclohexyle, cyclooctyle, cyclododécyle, phényle, naphtyle ou benzyle, et
R² signifie une simple liaison, C₁-C₂₀-alkylène, C₅-C₁₂-cycloalkylène, C₆-C₁₂-arylène ; ou C₂-C₂₀-alkylène interrompu par un ou plusieurs atomes d'oxygène et/ou de soufre et/ou par un ou plusieurs groupes imino substitués ou non substitués et/ou par un ou plusieurs groupes cycloalkyle, -(CO)-, -O(CO)O-, -(NH)(CO)O-, -O(CO)(NH)-, -O(CO)- ou -(CO)O-, les radicaux mentionnés pouvant à chaque fois être substitués par aryle, alkyle, aryloxy, alkyloxy, des hétéroatomes et/ou des hétérocycles,
où, pour le cas (2) dans la revendication 1, dans la formule II, R¹ représente hydrogène et au moins un des radicaux R³ à R¹⁰ est différent d'hydrogène.

2. Procédé selon l'une quelconque des revendications précédentes, **caractérisé en ce que** R² est choisi dans le groupe comprenant une simple liaison, méthylène, 1,1-éthylène, 1,2-éthylène, 1,1-propylène, 1,2-propylène, 1,3-propylène, 1,1-diméthyl-1,2-éthylène, 1,4-butylène, 1,6-hexylène, 2-méthyl-1,3-propylène, 2-éthyl-1,3-propylène, 2,2-diméthyl-1,3-propylène et 2,2-diméthyl-1,4-butylène, 3-méthyl-1,5-pentylène, 3,5-heptylène, 1,2-cyclopentylène, 1,3-cyclopentylène, 1,2-cyclohexylène, 1,3-cyclohexylène et ortho-phénylène.

3. Procédé selon l'une quelconque des revendications précédentes, **caractérisé en ce que** le polyalcool est choisi dans le groupe comprenant le 2-éthyl-1,3-hexanediol, le 2-méthyl-1,3-pentanediol, le 2-propyl-1,3-heptanediol, le 2,4-diéthyloctane-1,5-diol et le 2,2-diméthyl-1-phényl-1,3-propanediol.

4. Procédé selon l'une quelconque des revendications précédentes, **caractérisé en ce que** l'enzyme (E) est choisie parmi les estérases (E.C. 3.1.-.-), les lipases (E.C. 3.1.1.3), les glycosylases (E.C. 3.2.-.-) et les protéases (E.C. 3.4.-.-).
